# EUROPEAN PATENT SPECIFICATION

(11) **EP 4 116 302 B1**
(45) Date of publication and mention of the grant of the patent: **29.10.2025**
(21) Application number: 21905192.7
(22) Date of filing: 24.09.2021
(51) Int. Cl.: C07D 487/04, A61K 31/519, A61P 35/00

(54) **COMPOUND AND PREPARATION METHOD THEREOF AND APPLICATION THEREOF IN PREPARATION OF THERAPEUTIC ANTI-CANCER DRUG**
VERBINDUNG UND HERSTELLUNGSVERFAHREN DAFÜR UND ANWENDUNG DAVON BEI DER HERSTELLUNG EINES THERAPEUTISCHEN ANTIKREBSARZNEIMITTELS
COMPOSÉ, SON PROCÉDÉ DE PRÉPARATION ET SON UTILISATION DANS LA PRÉPARATION D'UN MÉDICAMENT ANTICANCÉREUX THÉRAPEUTIQUE

(30) Priority: 16.12.2020 CN 202011483614
(43) Date of publication of application: 11.01.2023
(73) Proprietor: Tianjin Jikun Medical Technology Co., Ltd., Tianjin 301700 (CN)
(72) Inventor: YANG, Cheng, Spring, Texas 77382 (US); YANG, Guang, Tianjin 301700 (CN); ZHOU, Honggang, Tianjin 301700 (CN); ZHANG, Liang, Tianjin 301700 (CN); LI, Jian, Tianjin 301700 (CN); LUN, Dongchao, Tianjin 301700 (CN)
(74) Representative: Giuliano, Natalia
(86) International application number: PCT/CN2021/120046
(87) International publication number: WO 2022/127261

(56) References cited:
- WO-A1-2013/102059
- WO-A1-2019/034075
- WO-A2-2008/039218
- CN-A- 106 928 231
- CN-A- 107 513 068
- CN-A- 109 970 740
- CN-A- 112 574 216
- CN-A- 113 264 937
- AOLI WANG ET AL: "Discovery of (R)-1-(3-(4-Amino-3-(3-chloro-4-(pyridin-2-ylmethoxy)phenyl)-1H-pyrazolo[3,4-d]pyrimidin-1-yl)piperidin-1-yl)prop-2-en-1-one (CHMFL-EGFR-202) as a Novel Irreversible EGFR Mutant Kinase Inhibitor with a Distinct Binding Mode", JOURNAL OF MEDICINAL CHEMISTRY, vol. 60, no. 7, 10 March 2017 (2017-03-10), US, pages 2944 - 2962, XP055405186, ISSN: 0022-2623, DOI: 10.1021/acs.jmedchem.6b01907
- WOLLE PATRIK ET AL: "Characterization of Covalent Pyrazolopyrimidine-MKK7 Complexes and a Report on a Unique DFG-in/Leu-in Conformation of Mitogen-Activated Protein Kinase Kinase 7 (MKK7)", JOURNAL OF MEDICINAL CHEMISTRY, vol. 62, no. 11, 14 May 2019 (2019-05-14), US, pages 5541 - 5546, XP093078018, ISSN: 0022-2623, DOI: 10.1021/acs.jmedchem.9b00472
- YIN YUAN ET AL: "Discovery of novel selective Janus kinase 2 (JAK2) inhibitors bearing a 1H-pyrazolo[3,4-d]pyrimidin-4-amino scaffold", BIOORGANIC & MEDICINAL CHEMISTRY, vol. 27, no. 8, 1 April 2019 (2019-04-01), AMSTERDAM, NL, pages 1562 - 1576, XP093078061, ISSN: 0968-0896, DOI: 10.1016/j.bmc.2019.02.054
- ENGEL JULIAN ET AL: "Structure-Guided Development of Covalent and Mutant-Selective Pyrazolopyrimidines to Target T790M Drug Resistance in Epidermal Growth Factor Receptor", JOURNAL OF MEDICINAL CHEMISTRY, vol. 60, no. 18, 18 September 2017 (2017-09-18), US, pages 7725 - 7744, XP093078033, ISSN: 0022-2623, DOI: 10.1021/acs.jmedchem.7b00515
- YUMING WANG ET AL: "Discovery of Potent Irreversible Pan-Fibroblast Growth Factor Receptor (FGFR) Inhibitors", JOURNAL OF MEDICINAL CHEMISTRY, vol. 61, no. 20, 9 March 2018 (2018-03-09), US, pages 9085 - 9104, XP055523638, ISSN: 0022-2623, DOI: 10.1021/acs.jmedchem.7b01843
- HE, LINHONG ET AL.: "Design, synthesis and biological evaluation of 7H-pyrrolo[2,3-d]pyrimidin-4-amine derivatives as selective Btk inhibitors with improved pharmacokinetic properties for the treatment of arthritis", EUROPEAN JOURNAL OF MEDICINAL CHEMISTRY, vol. 145, 28 December 2017 (2017-12-28), XP085414790, DOI: 10.1016/j.ejmech.2017.12.079

## Description

The present disclosure claims priority to the Chinese Patent Application 202011483614.1 filed to the China National Intellectual Property Administration (CNIPA) on December 16, 2020 and entitled "COMPOUND, PREPARATION METHOD THEREOF, AND USE THEREOF IN PREPARATION OF ANTI-CANCER DRUG".

### TECHNICAL FIELD

The present disclosure relates to the technical field of pharmacy, and in particular to a 2H-pyrazolo[3,4-d]pyrimidine derivative, a preparation method thereof, and use thereof in the preparation of an anti-cancer drug.

### BACKGROUND ART

Fibroblast growth factor receptors (FGFRs) are members of the receptor tyrosine kinase (RTK) family and are receptor protein tyrosine kinases (RPTKs), including four highly-conserved transmembrane tyrosine kinases: FGFR1, FGFR2, FGFR3, and FGFR4. A signaling pathway of FGFR is that a ligand binds to a receptor to induce FGFR dimerization, thereby causing cascade activation of downstream signaling pathways such as Ras-MAPK, PI3K-Akt, STAT, and PLCy. FGFRs play important roles in a variety of cell functions, such as cell proliferation and differentiation, and biological processes including development, angiogenesis, homeostasis, and wound repair. According to research surveys ⁽¹⁻³⁾, 7.1% of cancer patients have FGFR abnormalities. For example, the FGFR1 amplification occurred most in squamous non-small cell lung cancer (NSCLC) patients with a proportion of 20%, which was 10% in breast cancer patients, 5% in ovarian cancer patients, and 3% in bladder cancer patients. Currently, FGFR2 mutations have been found in gastric cancer (10%), endometrial cancer (12%), squamous NSCLC (5%), and triple-negative breast cancer (TNBC) (4%); and FGFR3 mutations are well known in bladder cancer (50% to 60%) and myeloma (15% to 20%). Aberrant FGF19/FGFR4 signaling is closely associated with hepatocellular carcinoma (HCC). Therefore, the development of a novel selective FGFR inhibitor to block the FGF/FGFR signaling pathway presents a promising therapeutic strategy for FGFR-associated cancer ⁽⁴⁻⁵⁾.

Researchers are committed to developing an FGFR inhibitor as a novel anti-cancer agent for the treatment of FGFR-associated cancer. Several multi-targeted tyrosine kinase inhibitors (TKIs) are initially used to treat FGFR abnormality-associated cancer⁽⁶⁾, such as dovitinib (TKI-258), lucitanib (E-3810)⁽⁷⁾, nintedanib (BIBF-1120)⁽⁸⁾, and ponatinib (AP-24534)⁽⁹⁾. These FGFR inhibitors developed early are non-selective⁽¹⁰⁾. Subsequently, several TKIs selective for FGFR with various scaffolds are developed. Some TKIs are currently in different stages of clinical research, such as AZD-4547 (phase III)⁽¹¹⁾, BGJ-398 (phase II)⁽¹²⁾, LY-2874455 (phase II)⁽¹³⁾, and JNJ-42756493 (phase II)⁽¹⁴⁾, and these selective FGFR inhibitors are ATP-competitive inhibitors that bind to an active form of FGFR. In addition, FGFR inhibitors developed by many pharmaceutical chemistry laboratories have been reported.⁽¹⁵⁻²¹⁾

AOLI WANG et al.⁽²²⁾ discloses: on the basis of Ibrutinib's core pharmacophore, which was moderately active to EGFR T790M mutant, we discovered novel epidermal growth factor receptor (EGFR) inhibitor compound 19 (CHMFL-EGFR-202), which potently inhibited EGFR primary mutants (L858R, del19) and drug-resistant mutant L858R/T790M. Compound 19 displayed a good selectivity profile among 468 kinases/mutants tested in the KINOMEscan assay (S score (1) = 0.02). In particular, it did not exhibit apparent activities against INSR and IGF1R kinases. The X-ray crystal structure revealed that this class of inhibitors formed a covalent bond with Cys797 in a distinct "DFG-in-C-helix-out" inactive EGFR conformation. Compound 19 displayed strong antiproliferative effects against EGFR mutant-driven nonsmall cell lung cancer (NSCLC) cell lines such as H1975, PC9, HCC827, and H3255 but not the wild-type EGFR expressing cells. In the H1975 and PC9 cell-inoculated xenograft mouse models, compound 19 exhibited dose-dependent tumor growth suppression efficacy without obvious toxicity. Compound 19 might be a potential drug candidate for EGFR mutant-driven NSCLC.

WOLLE PATRIK et al.⁽²³⁾ discloses: pyrazolopyrimidines are well-established as covalent inhibitors of protein kinases such as the epidermal growth factor receptor or Bruton's tyrosine kinase, and we recently described their potential in targeting mitogen-activated protein kinase kinase 7 (MKK7). Herein, we report the structure-activity relationship of pyrazolopyrimidine-based MKK7 inhibitors and solved several complex crystal structures to gain insights into their binding mode. In addition, we present two structures of apo-MKK7, exhibiting a DFG-out and an unprecedented DFG-in/Leu-in conformation.

YIN YUAN et al.⁽²⁴⁾ discloses: Janus kinases (JAKs) regulate various cancers and immune responses and are targets for the treatment of cancers and immune diseases. A new series of 1H-pyrazolo[3,4-d]pyrimidin-4-amino derivatives were synthesized and optimized by introducing a functional 3,5-disubstituted-1H-pyrazole moiety into the C-3 moiety of pyrazole template, and then were biologically evaluated as potent Janus kinase 2 (JAK2) inhibitors. Among these molecules, inhibitors 11f, 11g, 11h and 11k displayed strong activity and selectivity against the JAK2 kinase, with IC50 values of 7.2 nM, 6.5 nM, 8.0 nM and 9.7 nM, respectively. In particular, the cellular inhibitory assay and western blot analysis further support the JAK2 selectivity of compound 11g also in cells. Furthermore, compound 11g also exhibited potent inhibitory activity in lymphocytes proliferation assay and delayed hypersensitivity assay. Taken together, the novel JAK2 selective inhibitors discovered in this study may be potential lead compounds for new drug discovery via further development of more potent and selective JAK2 inhibitors.

WO2008/039218A2 discloses compounds that form covalent bonds with Bruton's tyrosine kinase (Btk). Also described are irreversible inhibitors of Btk. Methods for the preparation of the compounds are disclosed. Also disclosed are pharmaceutical compositions that include the compounds. Methods of using the Btk inhibitors are disclosed, alone or in combination with other therapeutic agents, for the treatment of autoimmune diseases or conditions, heteroimmune diseases or conditions, cancer, including lymphoma, and inflammatory diseases or conditions.

ENGEL JULIAN et al.⁽²⁵⁾ discloses reversible epidermal growth factor receptor (EGFR) inhibitors prompt a beneficial clinical response in non-small cell lung cancer patients who harbor activating mutations in EGFR. However, resistance mutations, particularly the gatekeeper mutation T790M, limit this efficacy. Here, we describe a structure-guided development of a series of covalent and mutant-selective EGFR inhibitors that effectively target the T790M mutant. The pyrazolopyrimidine-based core differs structurally from that of aminopyrimidine-based thirdgeneration EGFR inhibitors and therefore constitutes a new set of inhibitors that target this mechanism of drug resistance. These inhibitors exhibited strong inhibitory effects toward EGFR kinase activity and excellent inhibition of cell growth in the drug-resistant cell line H1975, without significantly affecting EGFR wild-type cell lines. Additionally, we present the in vitro ADME/DMPK parameters for a subset of the inhibitors as well as in vivo pharmacokinetics in mice for a candidate with promising activity profile.

YUMING WANG et al.⁽²⁶⁾ discloses: Fibroblast growth factor receptors (FGFR1-4) are promising therapeutic targets in many cancers. With the resurgence of interest in irreversible inhibitors, efforts have been directed to the discovery of irreversible FGFR inhibitors. Currently, several selective irreversible inhibitors are being evaluated in clinical trials that could covalently target a conserved cysteine in the P-loop of FGFR. In this article, we used a structure-guided approach that is rationalized by a computer-aided simulation to discover the novel and irreversible pan-FGFR inhibitor, 9g, which provided superior FGFR in vitro activities and decent selectivity over VEGFR2 (vascular endothelia growth factor receptor 2). In in vivo studies, 9g displayed clear antitumor activities in NCI-H1581 and SNU-16 xenograft mice models. Additionally, the diluting method confirmed the irreversible binding of 9g to FGFR.

CN113264937A discloses a 4-aminopyrazolo [3,4-d] pyrimidine derivative and application thereof, the structure of the derivative is as shown in formula I, A is an aromatic ring group or an aromatic heterocyclic group; L is fat alkane; Cyclic is of an N-containing heterocyclic structure; Q is carbonyl or sulfonyl; R is alkane, aryl or heteroaryl. A novel 4-aminopyrazolo [3,4-d] pyrimidine derivative is synthesized and screened, the activity of an ALK5 receptor can be inhibited in a dose-dependent mode, and the derivative or a solvate and a medicinal salt of the derivative can be applied to preparation of drugs for treating and/or preventing cancers.

CN109970740A discloses a 4-amino-pyrimidoazacycle derivative, a preparation method and application thereof. The 4-amino-pyrimidoazacycle derivative provided by the invention has a structural formula shown as formula X. In addition, the invention also provides a preparation method and application of the derivative. The 4-amino-pyrimidoazacyclederivative provided by the invention has the effect of inhibiting BTK.

HE Linhong et al. ⁽²⁷⁾ discloses: Bruton's tyrosine kinase (Btk) is a Tec family kinase with a well-defined role in the B cell receptor (BCR) and Fc γ receptor (FcR) signaling pathways, which makes it a uniquely attractive target for the treatment of autoimmune diseases, such as rheumatoid arthritis (RA). We reported a series of compounds bearing 7H-pyrrolo [2,3-d]pyrimidin-4-amine scaffold that potently inhibited Btk in vitro. Analysis of the structure-activity relationships (SAR) and drug-like profiles led to the discovery of the optimal compound B16. B16 preferentially inhibited Btk (IC50 = 21.70 ± 0.82 nM) over closely related kinases with moderate selectivity. Cell-based tests also confirmed that B16 significantly inhibited Btk Y223 autophosphorylation and PLC γ 2 Y1217 phosphorylation. MTT revealed that B16 displayed weak suppression against normal LO2, HEK293 and THP-1 cell lines with IC50 values over 30 µM. Moreover, B16 showed very weak potential to block the hERG channel (IC50 = 11.10 µM) in comparison to ibrutinib (IC50 = 0.97 µM). Owing to its favorable physicochemical properties (ClogP = 2.53, aqueous solubility ≈ 0.1 mg/mL), pharmacokinetic profiles (F = 49.15%, t1/2 = 7.02 h) and reasonable CYP450 profile, B16 exhibited potent anti-arthritis activity and similar efficacy to ibrutinib in reducing paw thickness in CIA mice. In conclusion, B16 is a potent, selective and durable inhibitor of Btk and has the potential to a safe and efficacious treatment for arthritis.

WO2013/102059A1 discloses compounds that form covalent bonds with Bruton's tyrosine kinase (Btk). Also described are irreversible inhibitors of Btk. Methods for the preparation of the compounds are disclosed. Also disclosed are pharmaceutical compositions that include the compounds. Methods of using the Btk inhibitors are disclosed, alone or in combination with other therapeutic agents, for the treatment of autoimmune diseases or conditions, heteroimmune diseases or conditions, cancer, including lymphoma, and inflammatory diseases or conditions.

CN107513068A discloses a novel compound having an FGFR inhibition activity, and a preparation method and an application thereof. The structure of the compound is represented by formula I, and all groups and substituent groups in the formula I are as defined in the description. The invention also discloses the preparation method of the compound, and a use of the compound in the preparation of drugs for treating and/or preventing tumor correlated diseases and/or FGFR correlated diseases.

WO2019/034075A1 discloses a FGFR and EGFR inhibitor, a compound represented by formula (I) and a pharmaceutically acceptable salt thereof. Also provided is the application of a drug for treating solid tumors, such as FGFR and EGFR related diseases.

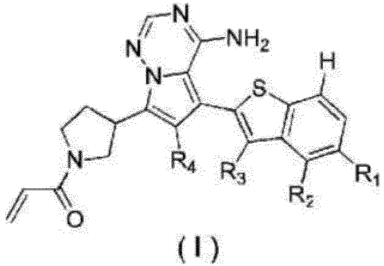

CN106928231A discloses a compound as shown in a formula I, namely a novel wild-type EGFR and/or mutant EGFR kinase inhibitor, which can be used for treating non-small cell lung cancer by single use or combined use with other therapeutic agents. The compound as shown in the formula I in the application can be used for treating patients with drug-resistance non-small cell lung cancer carrying wild-type EGFR and/or EGFR-T790M mutant and/or EGFR-T858R mutant and/or EGFR-delE746_A750 mutant.

### SUMMARY

The technical problems to be solved/objectives to be achieved by the present disclosure at least include: overcoming the defects in the prior art and providing a 2H-pyrazolo[3,4-d]pyrimidine derivative, a preparation method thereof, and use thereof in the preparation of an anti-cancer drug.

In order to achieve the above objectives, the present disclosure discloses the following technical solutions:
The present disclosure provides a compound of formula I or a pharmaceutically acceptable salt thereof: 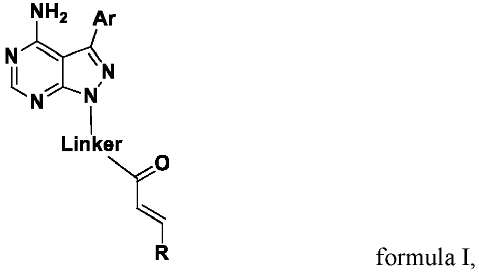
wherein the Ar is 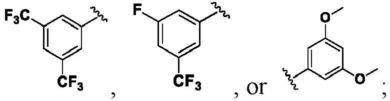
wherein the R is H, CF₃, (CH₃)₂NCH₂, or CH₃; and
wherein the Linker is 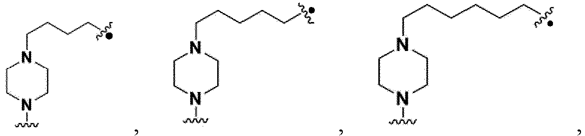 or 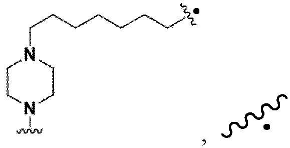 symbol representing a site bonding to N atom;
provided that the compound is not

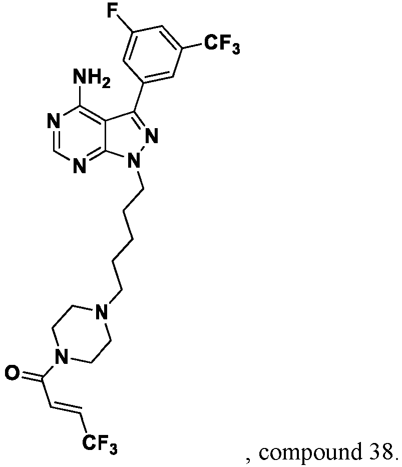

Preferably, the compound is one selected from the group consisting of the following compounds:

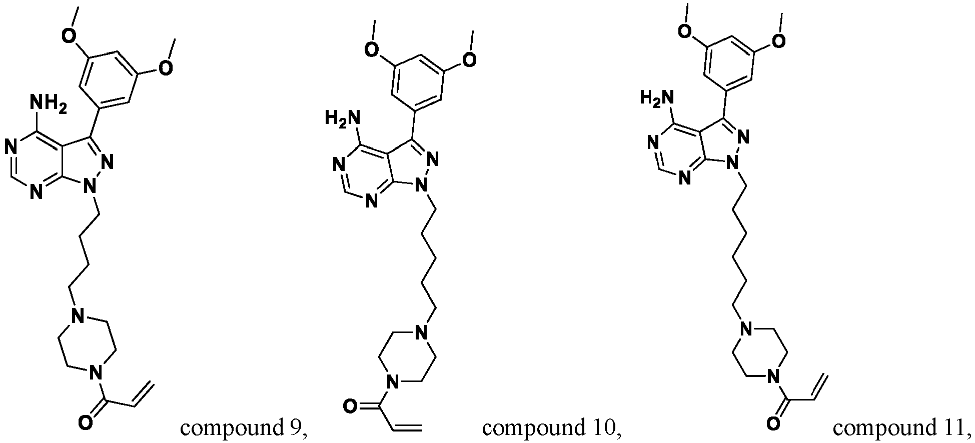

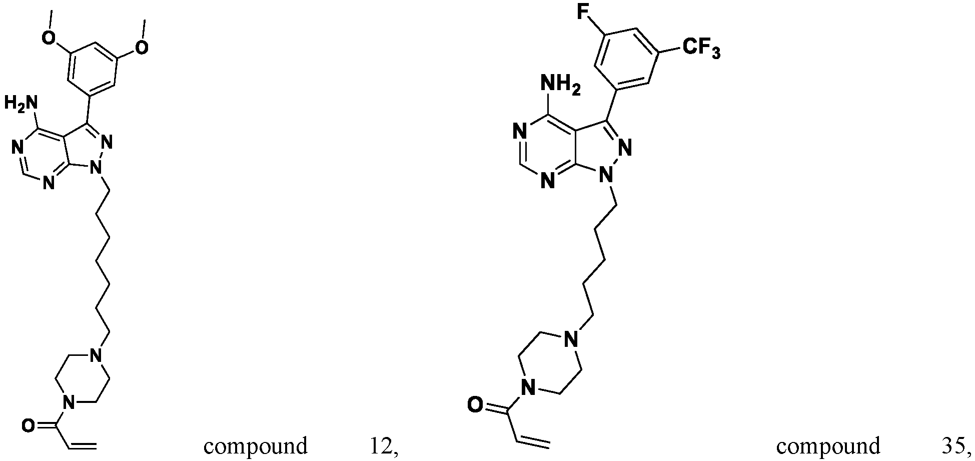

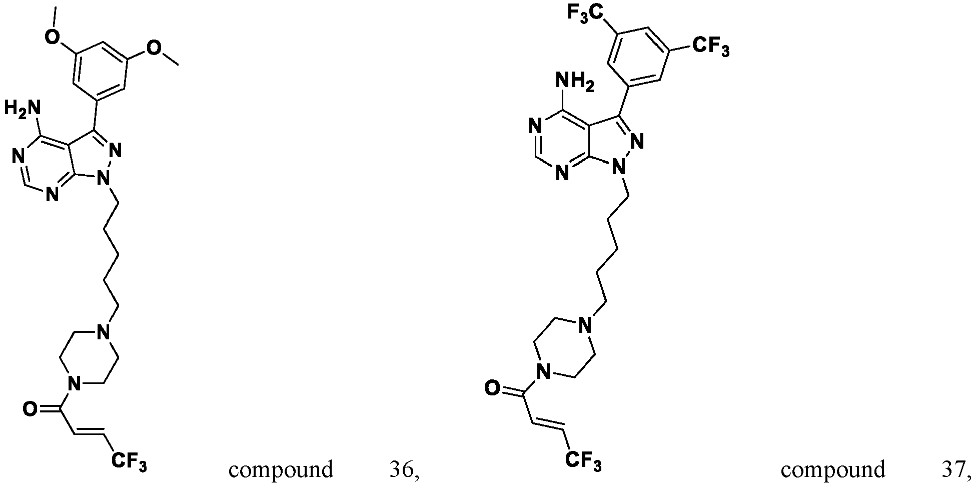

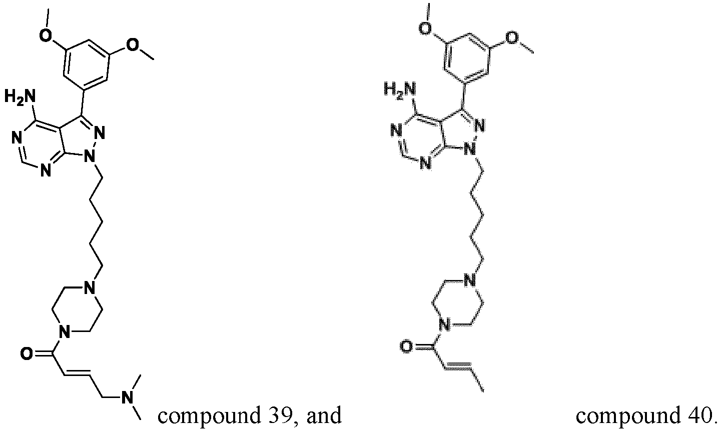

The present disclosure also provides a preparation method of the compound or the pharmaceutically acceptable salt thereof according to the above technical solution, with a synthetic route as follows:

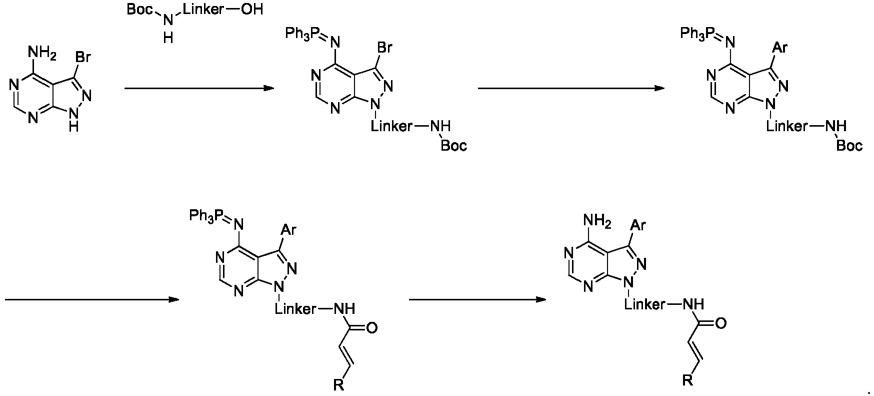

Preferably, the preparation method may include the following steps:
subjecting a compound with a structure shown in formula a and a compound with a structure shown in formula b to a Mitsunobu reaction at room temperature for 4 h to obtain a compound with a structure shown in formula c;
subjecting the compound with the structure shown in formula c and a compound with a structure shown in formula d to a Suzuki coupling reaction at 80°C to obtain a compound with a structure shown in formula e;
subjecting the compound with the structure shown in formula e and trifluoroacetic acid (TFA) to a deprotection reaction at 0°C to obtain a compound with a structure shown in formula f; and
subjecting the compound with the structure shown in formula f to an acylation reaction at room temperature to obtain the compound with a structure shown in formula I; 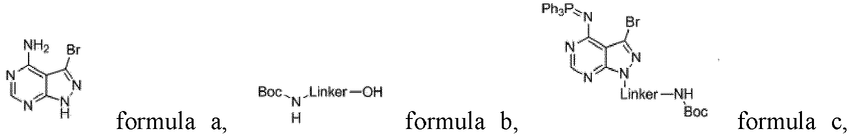 Ar-B(OH)₂ formula d,

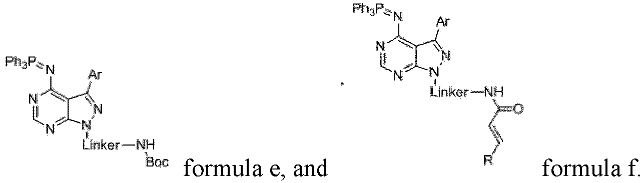

The present disclosure also provides use of the compound or the pharmaceutically acceptable salt thereof according to the above technical solution in the preparation of an tumor cell proliferation inhibitor, where a tumor cell is an FGFR abnormality-associated tumor cell.

Preferably, the tumor cell includes NCI-H1581 or SNU-16.

The present disclosure also provides use of the compound or the pharmaceutically acceptable salt thereof according to the above technical solution in blocking a fibroblast growth factor (FGF)/FGFR signaling pathway in a tumor cell, where the tumor cell is an FGFR abnormality-associated tumor cell.

Preferably, the tumor cell includes NCI-H1581 or SNU-16.

The present disclosure also provides a pharmaceutical composition including the compound or the pharmaceutically acceptable salt thereof according to the above technical solution, where the pharmaceutical composition further includes one or more pharmaceutically acceptable excipients; and
a dosage form of the pharmaceutical composition is any pharmaceutically acceptable dosage form.

The present disclosure also provides use of the compound or the pharmaceutically acceptable salt thereof according to the above technical solution or the pharmaceutical composition according to the above technical solution in the preparation of a drug for treating and/or preventing a cancer.

Preferably, the cancer is an FGFR abnormality-associated cancer.

The present disclosure also provides use of compound or the pharmaceutically acceptable salt thereof according to the above technical solution or the pharmaceutical composition according to the above technical solution for use in a method of treating and/or preventing a cancer.

The present disclosure also provides a method for treating a cancer, including the following step:
administering an anti-cancer drug to a patient through oral administration or intraperitoneal injection,
where the anti-cancer drug includes the compound or the pharmaceutically acceptable salt thereof according to the above technical solution.

Preferably, the anti-cancer drug includes the compound 10 or the compound 36.

Preferably, the anti-cancer drug is administered at a dose of 50 mg/kg or 100 mg/kg; and
the dose is calculated according to a dosage of the compound 10 or the compound 36.

Preferably, when the anti-cancer drug includes the compound 10, the anti-cancer drug is administered orally.

Preferably, when the anti-cancer drug includes the compound 36, the anti-cancer drug is administered through intraperitoneal injection.

In the present disclosure, the term "pharmaceutically acceptable adjuvant" includes pharmaceutically acceptable carriers, excipients, diluents, and the like, which are compatible with the active pharmaceutical ingredients. The preparation of a pharmaceutical formulation with a pharmaceutically acceptable adjuvant is well known to those of ordinary skill in the art.

In the present disclosure, the pharmaceutical composition may be combined with a pharmaceutically acceptable adjuvant (such as a carrier, an excipient, a diluent, or the like well known to those of ordinary skill in the art) to prepare various formulations, preferably including solid formulations and liquid preparations, such as tablets, pills, capsules, powders, suspensions, granules, syrups, emulsions, suspensions, and various sustained-release dosage forms, which may be preferably administered orally.

### BRIEF DESCRIPTION OF DRAWINGS

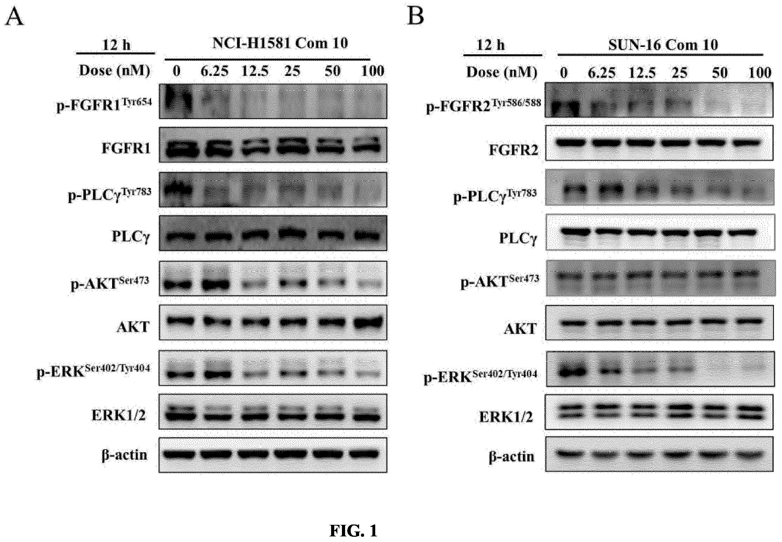

FIG. 1 shows that the compound 10 inhibits the activation of FGFR1 and a downstream signaling pathway thereof in NCI-H1581 cells and inhibits the activation of FGFR2 and a downstream signaling pathway thereof in SNU-16 cells in a concentration gradient-dependent manner, where A shows the inhibition on the activation of the FGFR1 and the downstream signaling pathway thereof in NCI-H1581 cells, and B shows the inhibition on the activation of the FGFR2 and the downstream signaling pathway thereof in SNU-16 cells;

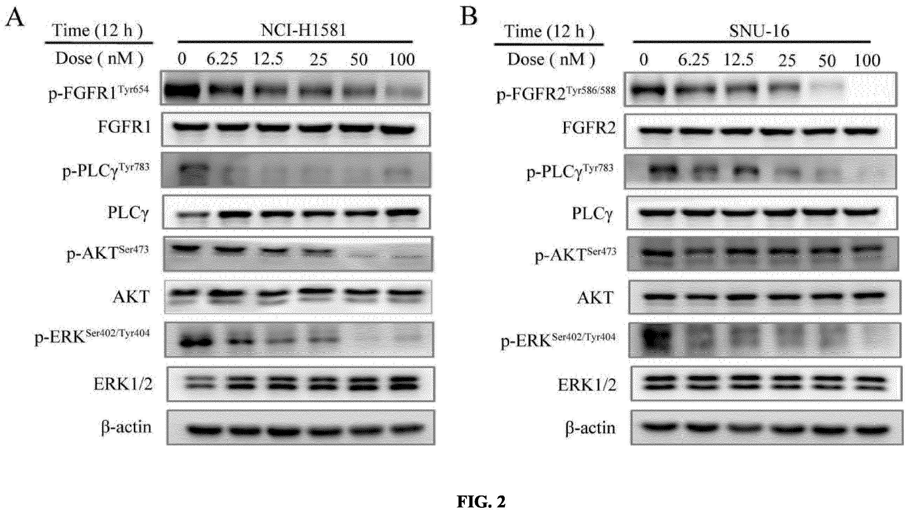

FIG. 2 shows that the compound 36 inhibits the activation of FGFR1 and a downstream signaling pathway thereof in NCI-H1581 cells and inhibits the activation of FGFR2 and a downstream signaling pathway thereof in SUN-16 cells in a concentration gradient-dependent manner, where A shows the inhibition on the activation of the FGFR1 and the downstream signaling pathway thereof in NCI-H1581 cells, and B shows the inhibition on the activation of the FGFR2 and the downstream signaling pathway thereof in SUN-16 cells;

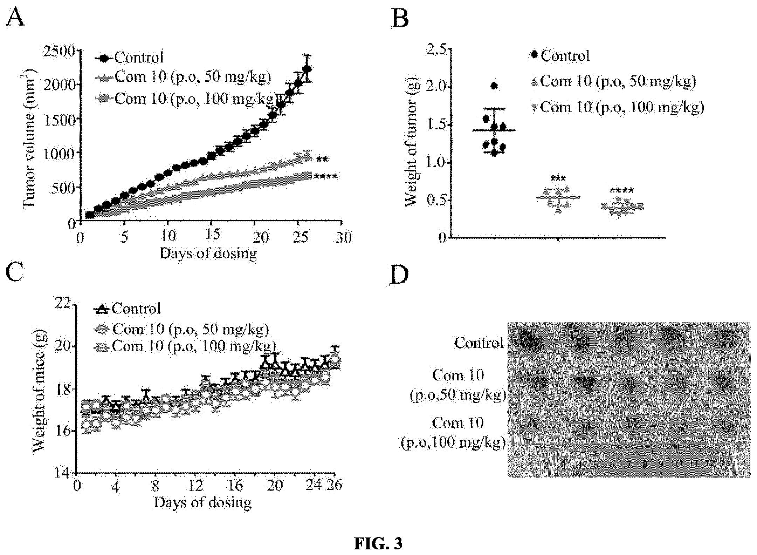

FIG. 3 shows tumor volume curves (A), a tumor weight statistics chart after 26 d of treatment (B), body weight change curves during a treatment process (C), and a picture of tumors after 26 d of treatment (D) in a tumor model subcutaneously transplanted with NCI-H1581 tumor cells under the tumor proliferation inhibition of the compound 10; and

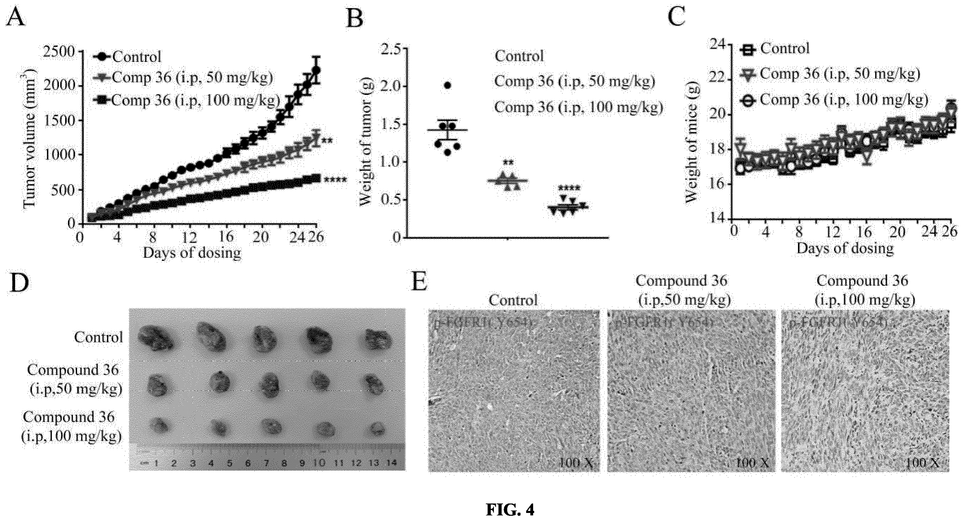

FIG. 4 shows tumor volume change curves (A), a tumor weight statistics chart after 26 d of treatment (B), body weight change curves during a treatment process (C), a picture of tumors after 26 d of treatment (D), and phosphorylation levels of FGFR1 in tissues of different treatments detected by immunohistochemistry (IHC) (E) in a tumor model subcutaneously transplanted with NCI-H1581 tumor cells under the tumor proliferation inhibition of the compound 36.

### DETAILED DESCRIPTION OF THE EMBODIMENTS

Unless otherwise defined, the technical terms used in the following examples have the same meanings as commonly understood by those skilled in the art to which the present disclosure belongs. Unless otherwise specified, in the following examples, the test reagents used are all conventional biochemical reagents, and the test methods are all conventional methods.

The present disclosure will be described in detail below with reference to the examples. Compounds 1-8, 13-33 and 38 are reference example only.

### Example 1: Preparation of compound 1

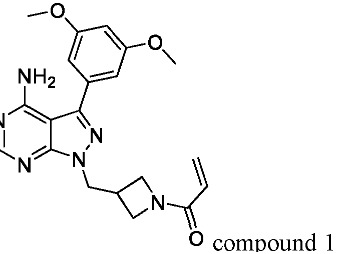

A specific preparation method was as follows:

### 1) Preparation of compound (1-1)

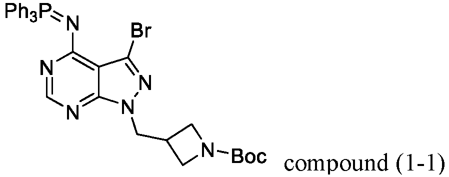

3-Bromo-1*H*-pyrazolo[3,4-d]pyrimidin-4-amine (500 mg, 2.34 mmol) was dissolved in dry tetrahydrofuran (THF) (23 mL), and then triphenylphosphine (TPP) (2.08 g, 7.94 mmol) and 1-Boc-3-azetidinemethanol were added; diisopropyl azodiformate (DIAD) (1.60 g, 7.94 mmol) was added dropwise at 0°C under the protection of argon, then the temperature was slowly raised to room temperature, and a reaction was further allowed for 4 h; and a reaction solution was spindried and purified by column chromatography (petroleum ether : ethyl acetate = 5:1 to 2:1) to obtain the compound 1-1 (yellow oily liquid, 685 mg, 69%).

Nuclear magnetic resonance (NMR) test results of the compound 1-1 were as follows:
¹H NMR (400 MHz, CDCl₃) *δ* 8.06 (s, 1H), 7.96 - 7.87 (m, 6H), 7.64 - 7.45 (m, 9H), 4.48 (d, *J* = 7.3 Hz, 2H), 3.97 (t, *J* = 8.5 Hz, 2H), 3.88 - 3.74 (m, 2H), 3.19 - 2.98 (m, 1H), 1.41 (s, 9H). ¹³C NMR (100 MHz, CDCl₃) *δ* 163.1, 163.0, 156.2, 155.9, 154.6, 154.5, 133.4, 133.4, 133.3, 133.3, 132.4, 132.3, 132.2, 128.8, 128.7, 128.7, 128.6, 128.6, 128.5, 128.4, 127.8, 127.4, 120.9, 107.1, 79.3, 52.9, 51.5, 49.7, 28.8, 28.4, 21.7, 14.2. HRMS (ESI) calculated for C₃₂H₃₂BrN₆NaO₂P⁺: 665.1400, found 665.1402.

### 2) Preparation of compound (1-2)

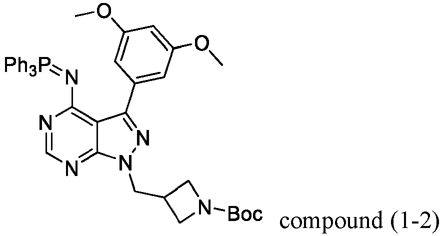

The compound 1-1 (5.01 mg, 6.74 mmol) and 3,5-dimethoxyphenylboronic acid (1.59 g, 8.76 mmol) were dissolved in a mixed solution of 1,4-dioxane and water (9 mL, v:v = 3:1), and then potassium carbonate (1.87 g, 13.5 mmol) and Pd(PPh₃)₄ (778 mg, 0.674 mmol) were added; a resulting system was argon replaced three times, and then heated to 80°C in an oil bath to undergo a reaction overnight; and after the reaction was completed, a resulting reaction system was washed with saturated sodium chloride (270 mL) and then subjected to extraction with ethyl acetate (90 mL), and a resulting organic phase was dried, concentrated, and purified by column chromatography (dichloromethane (DCM) : methanol = 70:1 to 30:1) to obtain the compound 1-2 (yellow oily liquid, 4.3 g, 60%).

NMR test results of the compound 1-2 were as follows:
¹H NMR (400 MHz, CDCl₃) *δ* 8.06 (d, *J =* 3.2 Hz, 1H), 7.89 - 7.71 (m, 6H), 7.56 - 7.40 (m, 11H), 6.70 - 6.41 (m, 1H), 4.58 (dd, *J =* 7.3, 3.2 Hz, 2H), 4.11 - 3.96 (m, 2H), 3.92 - 3.84 (m, 2H), 3.76 (d, *J* = 3.1 Hz, 6H), 3.26 - 3.07 (m, 1H), 1.41 (d, *J* = 3.1 Hz, 9H). ¹³C NMR (100 MHz, CDCl₃) *δ* 163.9, 163.9, 163.5, 163.4, 160.3, 156.3, 156.3, 155.2, 154.7, 146.1, 135.6, 134.2, 133.5, 133.4, 133.4, 133.3, 132.3, 132.2, 132.0, 132.0, 132.0, 1292, 128.6, 128.6, 128.5, 128.5, 128.4, 128.4, 128.2, 128.2, 127.8, 107.9, 100.6, 79.3, 77.4, 55.3, 49.5, 28.9, 28.4, 24.5. HRMS (ESI) calculated for C₄₀H₄₁N₆NaO₄P⁺: 723.2819, found 723.2822.

### 3) Preparation of compound (1-3)

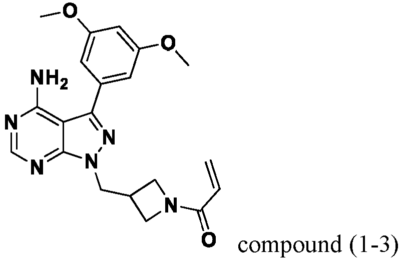

At 0°C, the compound 1-2 (450 mg, 0.587 mmol) was dissolved in DCM (5 mL), and then TFA (5 mL) was slowly added dropwise, after 30 min the reaction was completed; adjusting alkali of a resulting reaction solution was conducted with saturated NaHCO₃, and a resulting organic phase was concentrated to dry to obtain a brown-yellow oily intermediate. At 0°C, the intermediate was dissolved in 5 mL of DCM, then triethylamine (TEA) (98.2 µL, 0.704 mmol) was added, and then acryloyl chloride (52.2 µL, 0.646 mmol) was added dropwise; and a reaction was further allowed for 1 h at room temperature; and when it was monitored by thin layer chromatography (TLC) that the reaction was completed, a resulting reaction system was washed with saturated NaCl and then subjected to extraction with ethyl acetate, and a resulting organic phase was dried, concentrated, and purified by column chromatography (DCM : methanol = 30:1 to 10:1) to obtain the compound 1-3 (yellow oily liquid, 78 mg, 72%).

4) Preparation of compound 1-3: The compound 1-3 (100 mg, 0.189 mmol) was added to a mixed solution of acetic acid, THF, and water (3 mL, 3:1:2), and a reaction was conducted at room temperature for 18 h; and when it was monitored by TLC that the reaction was completed, a pH of a resulting reaction solution was adjusted to 7 to 8 with a saturated NaHCO₃ solution (15 mL), extraction was conducted with ethyl acetate (10 mL × 2), and a resulting organic phase was dried, concentrated, and purified by column chromatography (DCM : methanol = 15:1 to 7:1) to obtain the compound 1-3 (yellow solid, 57.8 mg, 70%).

NMR test results of the compound 1 were as follows:
¹H NMR (400 MHz, CDCl₃) *δ* 8.38 (s, 1H), 6.80 (d, *J* = 2.3 Hz, 2H), 6.57 (t, *J* = 2.3 Hz, 1H), 6.33 (dd, *J =* 17.0, 1.9 Hz, 1H), 6.17 (dd, *J =* 17.0, 10.3 Hz, 1H), 5.76 - 5.57 (m, 3H), 4.76 - 4.61 (m, 2H), 4.38 - 4.14 (m, 3H), 4.02 (dd, *J* = 10.5, 5.5 Hz, 1H), 3.87 (s, 6H), 3.49 - 3.25 (m, 1H). ¹³C NMR (100 MHz, CDCl₃) *δ* 165.7, 161.6, 157.8, 156.2, 154.7, 144.7, 134.8, 127.4, 127.2, 125.7, 106.3, 101.2, 98.4, 55.6, 53.5, 50.8, 49.4, 32.7, 29.1, 27.9. HRMS (ESI) calculated for C₂₀H₂₂N₆NaO₃⁺: 417.1646, found 417.1648.

### Example 2: Preparation of compound 2

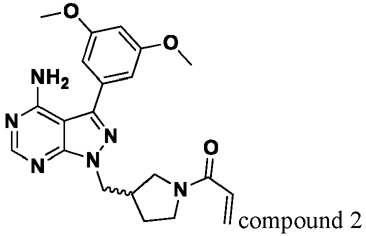

A specific preparation method was the same as the preparation method of compound 1, except that one of the raw materials was equimolar 1-Boc-3-hydroxymethylpyrrolidine, in replacement of 1-Boc-3-azetidinemethanol in the preparation method of compound (1-1). NMR test results of the compound 2 were as follows:
¹H NMR (400 MHz, CDCl₃) *δ* 8.38 (s, 1H), 6.81 (d, *J* = 2.2 Hz, 2H), 6.62 - 6.54 (m, 2H), 6.25 (dd, *J* = 16.9, 2.0 Hz, 1H), 5.76 (s, 2H), 5.66 (dd, *J* = 10.6, 2.0 Hz, 1H), 4.65 (d, *J* = 13.3 Hz, 1H), 4.49 (t, *J* = 7.2 Hz, 2H), 3.99 (d, *J* = 13.7 Hz, 1H), 3.86 (s, 6H), 3.00 (t, *J* = 12.6 Hz, 1H), 2.64 - 2.52 (m, 1H), 1.97 - 1.94 (m, 1H), 1.91 (s, 2H). ¹³C NMR (100 MHz, CDCl₃) *δ* 164.5, 161.5, 158.0, 156.0, 154.6, 144.6, 134.9, 128.5, 127.5, 106.4, 101.3, 98.3, 55.6, 50.0, 49.1, 45.7, 45.2, 39.9, 37.74 29.6, 27.9. HRMS (ESI) calculated for C₂₁H₂₄N₆NaO₃⁺: 431.1802, found 431.1804.

### Example 3: Preparation of compound 3

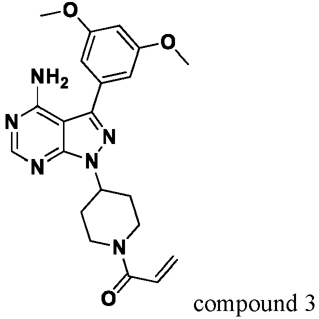

A specific preparation method was the same as the preparation method of compound 1, except that one of the raw materials was equimolar *N*-Boc-4-hydroxypiperidine, in replacement of 1-Boc-3-azetidinemethanol in the preparation method of compound (1-1). NMR test results of the compound 3 were as follows:
¹H NMR (400 MHz, CDCl₃) *δ* 8.38 (s, 1H), 6.80 (d, *J* = 2.2 Hz, 2H), 6.67 - 6.51 (m, 2H), 6.31 (dd, *J =* 16.8, 1.8 Hz, 1H), 5.84 - 5.63 (m, 3H), 5.05 (tt, *J* = 11.3, 4.2 Hz, 1H), 4.85 (d, *J* = 13.4 Hz, 1H), 4.20 (d, *J* = 13.9 Hz, 1H), 3.86 (s, 6H), 3.33 (t, *J* = 12.7 Hz, 1H), 3.02 - 2.85 (m, 1H), 2.34 (t, *J =* 11.8 Hz, 2H), 2.11 (d, *J* = 13.0 Hz, 2H). ¹³C NMR (101 MHz, CDCl₃) *δ* 161.5, 157.8, 155.8, 154.7, 154.3, 144.1, 135.1, 132.1, 131.6, 106.3, 101.1, 100.0, 98.3, 79.6, 55.7, 55.6, 52.9, 45.5, 29.7, 28.4, 26.8. HRMS (ESI) calculated for C₂₁H₂₄N₆NaO₃⁺: 431.1802, found 431.1805.

### Example 4: Preparation of compound 4

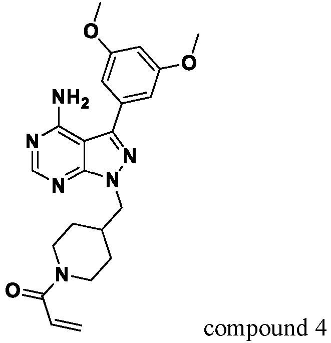

A specific preparation method was the same as the preparation method of compound 1, except that one of the raw materials was equimolar N-Boc-4-piperidinemethanol, in replacement of 1-Boc-3-azetidinemethanol in the preparation method of compound (1-1). NMR test results of the compound 4 were as follows:
¹H NMR (400 MHz, CDCl₃) *δ* 8.34 (s, 1H), 6.80 (d, *J* = 2.3 Hz, 2H), 6.57 - 6.49 (m, 2H), 6.23 (d, *J =* 16.8 Hz, 1H), 5.89 (s, 2H), 5.64 (d, *J =* 10.6 Hz, 1H), 4.65 (d, *J* = 13.3 Hz, 1H), 4.33 (d, *J* = 7.1 Hz, 2H), 3.97 (d, *J* = 13.8 Hz, 1H), 3.85 (s, 6H), 3.00 (t, *J* = 12.9 Hz, 1H), 2.62 (t, *J* = 12.9 Hz, 1H), 2.33 (d, *J =* 11.0 Hz, 1H), 1.66 (d, *J =* 16.3 Hz, 3H), 1.33 (td, *J* = 14.3, 7.5 Hz, 2H). ¹³C NMR (100 MHz, CDCl₃) *δ* 165.4, 161.5, 161.5, 157.9, 156.0, 154.7, 144.3, 134.9, 127.9, 127.9, 127.4, 106.4, 101.1, 99.9, 98.2, 55.6, 51.9, 45.6, 41.8, 36.8, 30.5, 29.4. HRMS (ESI) calculated for C₂₂H₂₆N₆NaO₃⁺: 445.1959, found 445.1961.

### Example 5: Preparation of compound 5

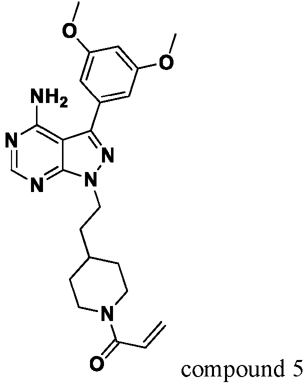

A specific preparation method was the same as the preparation method of compound 1, except that one of the raw materials was equimolar *N*-Boc-4-piperidineethanol, in replacement of 1-Boc-3-azetidinemethanol in the preparation method of compound (1-1). NMR test results of the compound 5 were as follows:
¹H NMR (400 MHz, CDCl₃) *δ* 8.38 (d, *J* = 1.1 Hz, 1H), 6.81 (d, *J* = 2.3 Hz, 2H), 6.57 (d, *J* = 2.4 Hz, 1H), 6.44 - 6.36 (m, 2H), 5.68 (dd, *J* = 9.6, 2.7 Hz, 1H), 4.50 (dd, *J* = 12.8, 6.9 Hz, 2H), 3.87 (s, 7H), 3.80 - 3.72 (m, 2H), 3.72 - 3.65 (m, 1H), 3.57 - 3.47 (m, 2H), 3.01 (d, *J* = 7.3 Hz, 1H), 1.65 (m, 4H). ¹³C NMR (100 MHz, CDCl₃) *δ* 166.3, 162.5, 158.7, 156.8, 155.2, 145.2, 136.0, 128.9, 128.4, 107.4, 102.1, 99.3, 56.6, 47.3, 45.6, 43.2, 36.9, 34.6, 33.5, 32.9, 32.6, 30.7, 30.4. HRMS (ESI) calculated for C₂₃H₂₈N₆NaO₃⁺: 459.2115, found 459.2115.

### Example 6: Preparation of compound 6

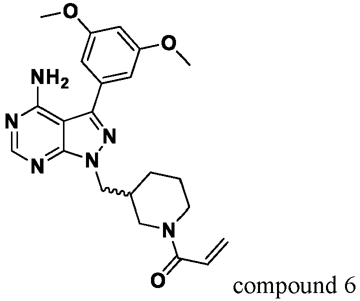

A specific preparation method was the same as the preparation method of compound 1, except that one of the raw materials was equimolar 1-Boc-3-hydroxymethylpiperidine, in replacement of 1-Boc-3-azetidinemethanol in the preparation method of compound (1-1). NMR test results of the compound 6 were as follows:
¹H NMR (400 MHz, CDCl₃) *δ* 8.39 - 8.33 (m, 1H), 6.93 - 6.75 (m, 2H), 6.63 - 6.46 (m, 2H), 6.21 (dd, *J =* 16.9, 8.7 Hz, 1H), 5.99 (s, 2H), 5.61 (dd, *J =* 32.0, 10.5 Hz, 1H), 4.53 - 4.27 (m, 3H), 3.86 (s, 6H), 3.07 (dt, *J =* 23.7, 12.4 Hz, 1H), 2.81 (dt, *J* = 44.0, 12.0 Hz, 1H), 2.35 (s, 1H), 1.93 (s, 1H), 1.58 - 1.29 (m, 2H), 1.26 (m, 2H). ¹³C NMR (100 MHz, CDCl₃) *δ* 165.5, 161.6, 157.9, 155.9, 154.7, 127.8, 127.4, 106.3, 101.3, 98.2, 55.6, 49.8, 49.7, 49.5, 46.5, 45.8, 42.7, 37.4, 36.4, 28.7, 25.2, 24.1. HRMS (ESI) calculated for C₂₂H₂₆N₆NaO₃⁺: 445.1959, found 445.1961.

### Example 7: Preparation of compound 7

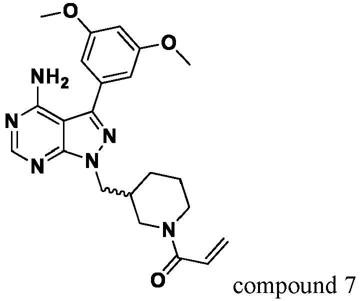

A specific preparation method was the same as the preparation method of compound 1, except that one of the raw materials was equimolar tert-Butyl 4-(2-hydroxyethyl)piperazine-1-carboxylate, in replacement of 1-Boc-3-azetidinemethanol in the preparation method of compound (1-1). NMR test results of the compound 7 were as follows:
¹H NMR (400 MHz, CDCl₃) *δ* 8.36 (s, 1H), 6.80 (d, *J* = 2.3 Hz, 2H), 6.60 - 6.47 (m, 2H), 6.26 (dd, *J* = 16.8, 1.9 Hz, 1H), 5.67 (dd, *J* = 10.5, 1.9 Hz, 1H), 4.57 (t, *J* = 6.7 Hz, 2H), 3.85 (s, 6H), 3.62 (s, 2H), 3.49 (s, 2H), 2.96 (t, *J* = 6.7 Hz, 2H), 2.56 (t, *J* = 5.0 Hz, 4H). ¹³C NMR (100 MHz, CDCl₃) *δ* 165.3, 161.5, 157.4, 155.1, 154.5, 144.6, 134.8, 127.9, 127.4, 106.3, 101.2, 98.2, 95.6, 56.7, 55.6, 52.6, 50.9, 45.6, 44.4, 41.8, 31.9, 29.1. HRMS (ESI) calculated for C₂₂H₂₇N₇NaO₃⁺: 460.2068, found 460.2072.

### Example 8: Preparation of compound 8

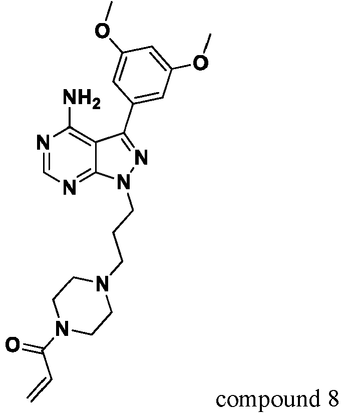

A specific preparation method was the same as the preparation method of compound 1, except that one of the raw materials was equimolar 1-Boc-4-(3-hydroxypropane)piperazine, in replacement of 1-Boc-3-azetidinemethanol in the preparation method of compound (1-1). NMR test results of the compound 8 were as follows:
¹H NMR (400 MHz, CDCl₃) δ 8.36 (s, 1H), 6.81 (d, *J* = 2.3 Hz, 2H), 6.58 - 6.50 (m, 2H), 5.88 (s, 2H), 5.69 (dd, *J* = 10.5, 1.9 Hz, 1H), 4.52 (t, *J* = 6.9 Hz, 2H), 3.86 (s, 6H), 3.69 (d, *J* = 6.9 Hz, 2H), 3.57 (s, 2H), 2.51 (dt, *J* = 10.8, 6.0 Hz, 6H), 2.21 (p, *J* = 7.0 Hz, 2H). ¹³C NMR (100 MHz, CDCl₃) *δ* 165.3, 160.3, 154.4, 133.4, 133.3, 132.2, 132.1, 132.0, 129.0, 128.6, 128.6, 128.4, 128.1, 127.3, 127.3, 107.9, 100.5, 55.4, 55.3, 53.1, 52.5, 45.2, 26.4. HRMS (ESI) calculated for C₂₃H₂₉N₇NaO₃⁺: 474.2224, found 474.2226.

### Example 9: Preparation of compound 9

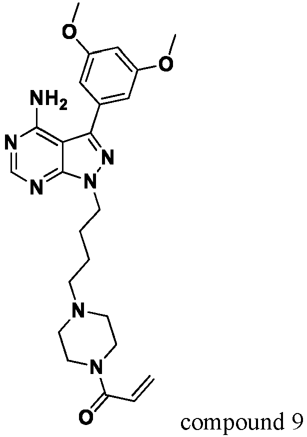

A specific preparation method was the same as the preparation method of compound 1, except that one of the raw materials was equimolar 1-Boc-4-(4-hydroxybutane)piperazine, in replacement of 1-Boc-3-azetidinemethanol in the preparation method of compound (1-1). NMR test results of the compound 9 were as follows:
¹H NMR (400 MHz, CDCl₃) *δ* 8.40 - 8.36 (m, 1H), 6.81 (dd, *J* = 3.2, 2.0 Hz, 2H), 6.54 (ddd, *J* = 17.4, 8.7, 4.1 Hz, 2H), 6.28 (dt, *J =* 16.9, 2.5 Hz, 1H), 5.72 - 5.66 (m, 1H), 5.61 (s, 2H), 4.46 (s, 2H), 3.89 - 3.82 (m, 6H), 3.67 (s, 2H), 3.54 (s, 2H), 2.41 (s, 6H), 2.01 (s, 2H), 1.56 (s, 2H). ¹³C NMR (100 MHz, MeOD) *δ* 169.4, 166.0, 152.2, 151.70, 147.6, 145.9, 132.7, 128.5, 126.6, 106.2, 106.1, 101.5, 101.4, 96.5, 56.6, 54.7, 53.5, 51.1, 40.5, 39.1, 38.5, 35.2, 28.3, 22.7. HRMS (ESI) calculated for C₂₄H₃₁N₇NaO₃⁺: 488.2381, found 488.2383.

### Example 10: Preparation of compound 10

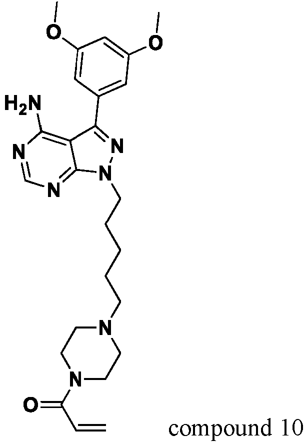

A specific preparation method was the same as the preparation method of compound 1, except that one of the raw materials was equimolar 1-Boc-4-(5-hydroxypentane)piperazine, in replacement of 1-Boc-3-azetidinemethanol in the preparation method of compound (1-1). NMR test results of the compound 10 were as follows:
¹H NMR (400 MHz, CDCl₃) *δ* 8.38 (s, 1H), 6.82 - 6.80 (m, 2H), 6.58 - 6.55 (m, 1H), 6.53 (d, *J* = 10.4 Hz, 1H), 6.27 (d, *J* = 16.7 Hz, 1H), 5.68 (d, *J* = 10.6 Hz, 1H), 5.61 (s, 2H), 4.43 (s, 2H), 3.86 (s, 6H), 3.67 (s, 2H), 3.53 (s, 2H), 2.42 - 2.38 (m, 4H), 2.34 - 2.30 (m, 2H), 2.02 - 1.96 (m, 2H), 1.54 (d, *J* = 7.4 Hz, 2H), 1.39 (d, *J* = 6.9 Hz, 2H). ¹³C NMR (100 MHz, CDCl₃) *δ* 165.3, 161.6, 157.7, 155.9, 154.3, 144.0, 135.1, 127.7, 127.5, 106.4, 101.1, 100.0, 98.3, 58.2, 55.6, 53.4, 52.7, 47.1, 45.7, 41.8, 40.5, 29.6, 29.3, 26.2, 24.5. HRMS (ESI) calculated for C₂₅H₃₃N₇NaO₃⁺: 502.2537, found 502.2539.

### Example 11: Preparation of compound 11

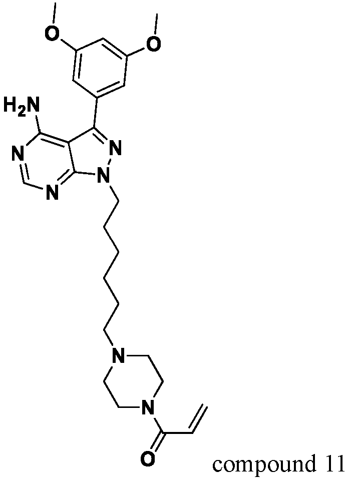

A specific preparation method was the same as the preparation method of compound 1, except that one of the raw materials was equimolar 1-Boc-4-(6-hydroxyhexane)piperazine, in replacement of 1-Boc-3-azetidinemethanol in the preparation method of compound (1-1). NMR test results of the compound 11 were as follows:
¹H NMR (400 MHz, CDCl₃) *δ* 8.34 (d, *J =* 1.3 Hz, 1H), 6.81 (dd, *J* = 2.3, 1.3 Hz, 2H), 6.61 - 6.52 (m, 2H), 6.29 (dt, *J* = 16.9, 1.6 Hz, 1H), 6.00 (s, 2H), 5.69 (dt, *J =* 10.5, 1.6 Hz, 1H), 4.43 (t, *J* = 7.3 Hz, 2H), 3.86 (d, *J* = 1.3 Hz, 6H), 3.69 (t, *J* = 5.0 Hz, 2H), 3.56 (t, *J* = 4.9 Hz, 2H), 2.42 (t, *J* = 5.0 Hz, 4H), 2.33 (t, *J* = 7.6 Hz, 2H), 1.97 (t, *J* = 7.2 Hz, 2H), 1.54 - 1.45 (m, 2H), 1.38 (t, *J* = 4.3 Hz, 4H). ¹³C NMR (100 MHz, CDCl₃) *δ* 165.3, 161.5, 157.8, 155.3, 154.1, 144.2, 135.0, 135.0, 127.9, 127.5, 127.5, 106.4, 106.4, 101.1, 98.2, 58.3, 55.6, 53.4, 52.7, 47.2, 45.7, 41.8, 29.7, 27.0, 26.6, 26.5. HRMS (ESI) calculated for C₂₆H₃₅N₇NaO₃⁺: 516.2694, found 516.2696.

### Example 12: Preparation of compound 12

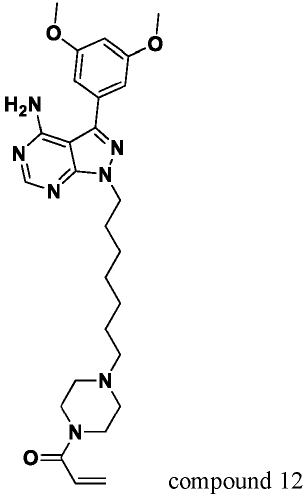

A specific preparation method was the same as the preparation method of compound 1, except that one of the raw materials was equimolar 1-Boc-4-(7-hydroxyheptane)piperazine, in replacement of 1-Boc-3-azetidinemethanol in the preparation method of compound (1-1). NMR test results of the compound 12 were as follows:
¹H NMR (400 MHz, CDCl₃) *δ* 8.38 (d, *J* = 2.8 Hz, 1H), 6.82 (t, *J* = 2.5 Hz, 2H), 6.62 - 6.50 (m, 2H), 6.33 - 6.23 (m, 1H), 5.77 - 5.56 (m, 3H), 4.42 (s, 2H), 3.86 (d, *J* = 2.8 Hz, 6H), 3.70 (s, 2H), 3.57 (s, 2H), 2.50 - 2.37 (m, 4H), 2.33 (s, 2H), 1.96 (s, 2H), 1.47 (s, 2H), 1.33 (d, *J* = 27.0 Hz, 6H). ¹³C NMR (101 MHz, CDCl₃) *δ* 165.6, 161.8, 158.1, 156.1, 154.5, 144.3 135.5, 135.5, 128.1, 127.8, 106.7, 101.4, 101.4, 98.6, 58.7, 55.9, 53.7, 53.1, 47.5, 46.0, 42.2, 30.0, 29.9, 29.3, 27.6, 26.9, 26.8. HRMS (ESI) calculated for C₂₇H₃₇N₇NaO₃⁺: 530.2850, found 530.2852.

### Example 13: Preparation of compound 13

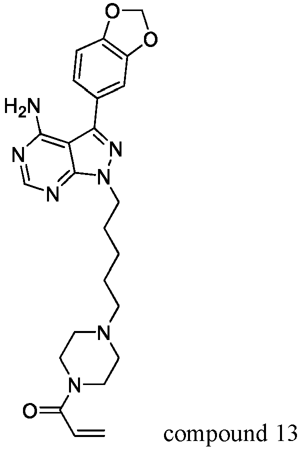

A specific preparation method was the same as the preparation method of compound 1, except that one of the raw materials was equimolar 3,4-methylenephenylboronic acid, in replacement of 3,5-dimethoxyphenylboronic acid in the preparation method of compound (1-2). NMR test results of the compound 13 were as follows:
¹H NMR (400 MHz, CDCl₃) *δ* 8.36 (s, 1H), 7.16 (d, *J* = 2.7 Hz, 1H), 6.97 (d, *J* = 7.9 Hz, 1H), 6.62 - 6.49 (m, 1H), 6.28 (dd, *J* = 16.9, 2.0 Hz, 1H), 6.06 (s, 2H), 5.86 - 5.65 (m, 3H), 4.42 (t, *J* = 7.2 Hz, 2H), 3.70 (d, *J* = 7.5 Hz, 2H), 3.55 (t, *J* = 5.1 Hz, 2H), 2.43 (t, *J* = 4.9 Hz, 4H), 2.35 (t, *J =* 7.6 Hz, 2H), 1.99 (td, *J* = 16.9, 15.0, 9.4 Hz, 2H), 1.79 - 1.14 (m, 6H). ¹³C NMR (100 MHz, CDCl₃) *δ* 165.2, 157.8, 155.7, 154.2, 148.6, 148.5, 143.8, 127.4, 127.1, 122.1, 109.0, 108.9, 101.6, 101.5, 98.2, 58.1, 53.3, 52.7, 46.9, 45.6, 41.7, 29.5, 26.1, 24.5. HRMS (ESI) calculated for C₂₄H₂₉N₇NaO₃⁺: 4860.2224, found 4860.2226.

### Example 14: Preparation of compound 14

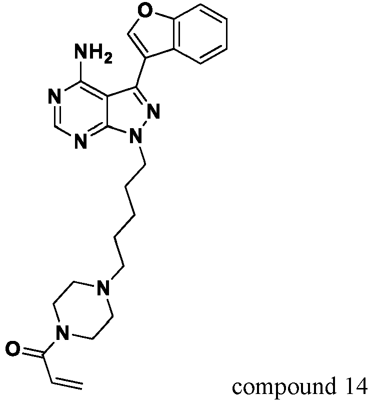

A specific preparation method was the same as the preparation method of compound 1, except that one of the raw materials was equimolar benzofuran-2-boronic acid, in replacement of 3,5-dimethoxyphenylboronic acid in the preparation method of compound (1-2). NMR test results of the compound 14 were as follows:
¹H NMR (400 MHz, CDCl₃) *δ* 8.39 (s, 1H), 7.68 (dd, *J* = 7.3, 1.5 Hz, 1H), 7.59 - 7.55 (m, 1H), 7.40 - 7.32 (m, 3H), 6.53 (dd, *J* = 16.8, 10.6 Hz, 1H), 6.28 (dd, *J* = 16.9, 1.9 Hz, 1H), 5.68 (dd, *J =* 10.6, 2.0 Hz, 1H), 4.46 (t, *J* = 7.2 Hz, 2H), 3.69 (s, 2H), 3.55 (s, 2H), 2.44 (s, 4H), 2.36 (t, *J* = 7.6 Hz, 2H), 2.01 (p, *J* = 7.4 Hz, 2H), 1.57 (q, *J* = 7.6 Hz, 2H), 1.44 - 1.36 (m, 2H). ¹³C NMR (100 MHz, CDCl₃) *δ* 165.3, 163.8, 157.6, 156.0 154.4, 149.5, 134.7, 128.3, 127.8, 127.4, 125.1, 124.0, 121.7, 111.0, 104.3, 97.9, 58.1, 53.3, 52.7, 47.3, 45.6, 41.7, 29.5, 26.1, 24.4. HRMS (ESI) calculated for C₂₅H₂₉N₇NaO₂⁺: 482.2269, found 482.2271.

### Example 15: Preparation of compound 15

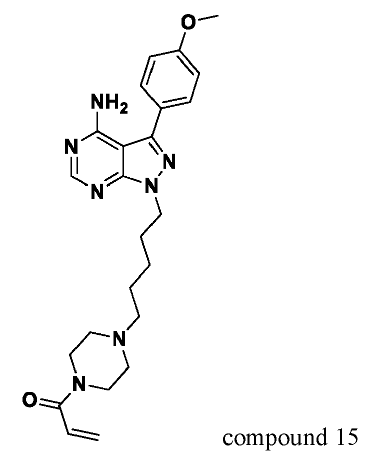

A specific preparation method was the same as the preparation method of compound 1, except that one of the raw materials was equimolar *p*-methoxyboronic acid, in replacement of 3,5-dimethoxyphenylboronic acid in the preparation method of compound (1-2). NMR test results of the compound 15 were as follows:
¹H NMR (400 MHz, MeOD) *δ* 8.24 (s, 1H), 7.61 (d, *J* = 8.6 Hz, 2H), 7.18 - 7.06 (m, 2H), 6.82 - 6.64 (m, 1H), 6.19 (dd, *J =* 16.7, 2.0 Hz, 1H), 5.74 (dd, *J =* 10.6, 1.9 Hz, 1H), 4.41 (t, *J* = 6.8 Hz, 2H), 3.88 (s, 3H), 3.62 (s, 4H), 2.48 (s, 4H), 2.39 (s, 2H), 1.96 (q, *J* = 7.3 Hz, 2H), 1.60 - 1.52 (m, 2H), 1.35 (q, *J =* 7.9 Hz, 2H). ¹³C NMR (100 MHz, CDCl₃) *δ* 165.3, 157.7, 155.9, 155.9, 154.4, 143.6, 140.4, 133.9, 132.5, 129.6, 127.8, 126.7, 125.9, 124.7, 64.1, 58.2, 53.4, 52.7, 48.6, 47.1, 29.5, 26.0, 24.5, 15.5. HRMS (ESI) calculated for C₂₄H₃₁N₇NaO₂⁺: 472.2426, found 472.2428.

### Example 16: Preparation of compound 16

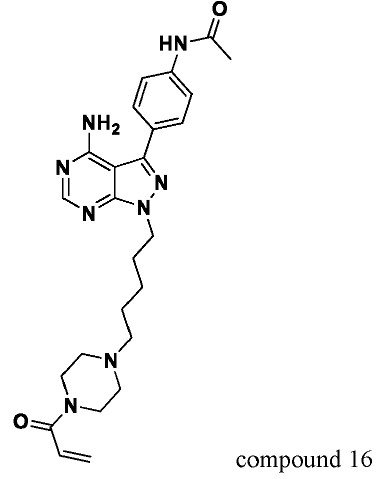

A specific preparation method was the same as the preparation method of compound 1, except that one of the raw materials was equimolar 4-acetylaminophenylboronic acid, in replacement of 3,5-dimethoxyphenylboronic acid in the preparation method of compound (1-2). NMR test results of the compound 16 were as follows:
¹H NMR (400 MHz, CDCl₃) *δ* 8.24 (d, *J =* 1.3 Hz, 1H), 8.01 (s, 1H), 7.54 - 7.43 (m, 3H), 6.72 (dd, *J* = 16.9, 10.7 Hz, 1H), 6.18 (dt, *J* = 16.8, 1.7 Hz, 1H), 5.73 (dt, *J* = 10.6, 1.7 Hz, 1H), 4.43 (t, *J =* 6.7 Hz, 2H), 3.60 (q, *J* = 6.7, 4.6 Hz, 4H), 2.46 (d, *J =* 5.2 Hz, 4H), 2.37 (t, *J =* 7.6 Hz, 2H), 2.16 (d, *J =* 1.4 Hz, 3H), 1.97 (t, *J =* 7.4 Hz, 2H), 1.57 (dd, *J =* 11.0, 4.7 Hz, 2H), 1.34 (p, *J* = 7.6 Hz, 2H). ¹³C NMR (100 MHz, MeOD) *δ* 170.6, 165.9, 158.4, 155.4, 153.8, 144.4, 139.14, 133.2, 129.6, 127.3, 127.3, 123.7, 120.4, 120.1, 100.0, 97.6, 57.6, 52.8, 52.3, 45.0, 41.3, 28.9, 25.3, 23.9, 22.4. HRMS (ESI) calculated for C₂₅H₃₂N₈NaO₂⁺: 472.2426, found 472.2428.

### Example 17: Preparation of compound 17

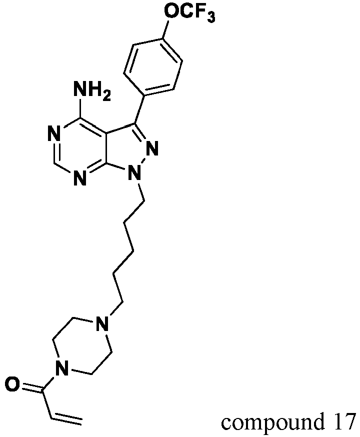

A specific preparation method was the same as the preparation method of compound 1, except that equimolar 4-trifluoromethoxyphenylboronic acid, in replacement of 3,5-dimethoxyphenylboronic acid in the preparation method of compound (1-2). NMR test results of the compound 17 were as follows:
¹H NMR (400 MHz, CDCl₃) δ 8.27 (s, 1H), 7.70 (t, *J* = 7.6 Hz, 1H), 7.63 (d, *J* = 10.6 Hz, 1H), 7.49 - 7.42 (m, 1H), 7.35 (dd, *J* = 7.8*,* 5.3 Hz, 1H), 6.73 (dd, *J* = 16.8, 10.7 Hz, 1H), 6.18 (dd, *J* = 16.8, 1.9 Hz, 1H), 5.73 (dd, *J* = 10.7, 2.0 Hz, 1H), 4.44 (t, *J* = 6.8 Hz, 2H), 3.61 (d, *J* = 5.4 Hz, 4H), 2.50 - 2.37 (m, 4H), 2.35 (d, *J* = 7.7 Hz, 2H), 1.99 (q, *J* = 7.1 Hz, 2H), 1.59 - 1.55 (m, 2H), 1.35 (s, 2H). ¹³C NMR (100 MHz, CDCl₃) *δ* 166.2, 158.5, 156.8, 155.4, 150.6, 143.3, 136.3, 131.8, 128.6, 128.4, 127.6, 122.3, 121.9, 120.1, 99.2, 59.1, 54.3, 53.6, 48.1, 46.6, 42.8, 30.4, 27.1, 25.4. HRMS (ESI) calculated for C₂₄H₂₈F₃N₇NaO₂⁺: 526.2143, found 526.2145.

### Example 18: Preparation of compound 18

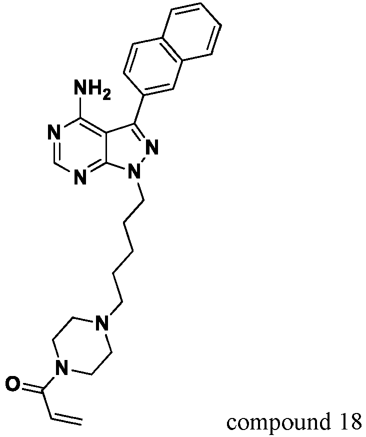

A specific preparation method was the same as the preparation method of compound 1, except that one of the raw materials was equimolar 2-naphthaleneboronic acid in replacement of 3,5-dimethoxyphenylboronic acid in the preparation method of compound (1-2). NMR test results of the compound 18 were as follows:
¹H NMR (400 MHz, CDCl₃) δ 8.41 (s, 1H), 8.17 (s, 1H), 8.03 (d, *J* = 8.4 Hz, 1H), 7.93 (dd, *J* = 9.3*,* 3.6 Hz, 2H), 7.82 (d, *J =* 8.4 Hz, 1H), 7.58 (dd, *J* = 6.2, 3.2 Hz, 2H), 6.51 (dd, *J* = 16.8, 10.5 Hz, 1H), 6.27 (d, *J* = 16.8 Hz, 1H), 5.67 (d, *J* = 10.7 Hz, 1H), 4.49 (t, *J* = 7.2 Hz, 2H), 3.68 (s, 2H), 3.51 (d, *J =* 15.2 Hz, 2H), 2.37 (dd, *J =* 19.5, 12.3 Hz, 6H), 2.04 (dd, *J =* 14.6, 7.4 Hz, 2H), 1.57 (dd, *J* = 14.7, 7.5 Hz, 2H), 1.42 (dd, *J* = 14.9, 7.9 Hz, 2H). ¹³C NMR (100 MHz, CDCl₃) δ 165.28, 157.63, 155.97, 154.54, 149.88, 142.42, 135.37, 135.35, 130.91, 127.75, 127.50, 127.24, 126.68, 121.64, 121.38, 121.00, 118.73, 98.31, 58.16, 53.43, 52.73, 47.17, 45.72, 41.87, 29.53, 26.24, 24.52. HRMS (ESI) calculated for C₂₇H₃₁N₇NaO+: 492.2482, found 492.2484.

### Example 19: Preparation of compound 19

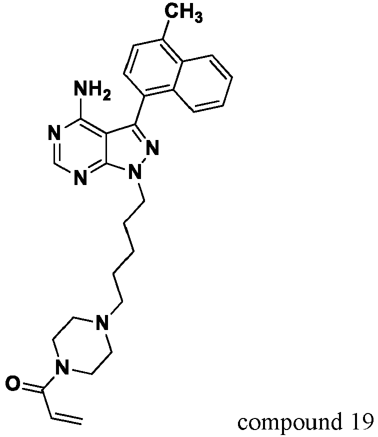

A specific preparation method was the same as the preparation method of compound 1, except that one of the raw materials was equimolar 4-methylnaphthaleneboronic acid, in replacement of 3,5-dimethoxyphenylboronic acid in the preparation method of compound (1-2). NMR test results of the compound 19 were as follows:
¹H NMR (400 MHz, CDCl₃) δ 8.37 (s, 1H), 8.10 (d, *J* = 8.4 Hz, 1H), 7.91 (d, *J* = 8.3 Hz, 1H), 7.63 - 7.41 (m, 4H), 6.52 (dd, *J =* 16.8, 10.5 Hz, 1H), 6.26 (dd, *J* = 16.8, 1.7 Hz, 1H), 5.67 (dd, *J =* 10.5, 1.7 Hz, 1H), 4.50 (t, *J* = 7.1 Hz, 2H), 3.66 (s, 2H), 3.49 (d, *J* = 16.9 Hz, 2H), 2.78 (s, 3H), 2.36 (dd, *J* = 19.3, 12.0 Hz, 6H), 2.13 - 1.98 (m, 2H), 1.57 (dt, *J* = 14.9, 7.6 Hz, 2H), 1.42 (dt, *J =* 15.0, 7.6 Hz, 2H). ¹³C NMR (100 MHz, CDCl₃) δ 165.27, 159.72, 157.75, 155.67, 154.23, 144.16, 134.49, 130.52, 127.93, 127.88, 127.43, 120.40, 120.11, 115.46, 114.30, 98.54, 98.31, 63.68, 58.15, 53.31, 52.69, 47.03, 41.74, 29.71, 29.54, 26.11, 24.50, 24.04. HRMS (ESI) calculated for C₂₈H₃₃N₇NaO+: 570.1587, found 570.1590.

### Example 20: Preparation of compound 20

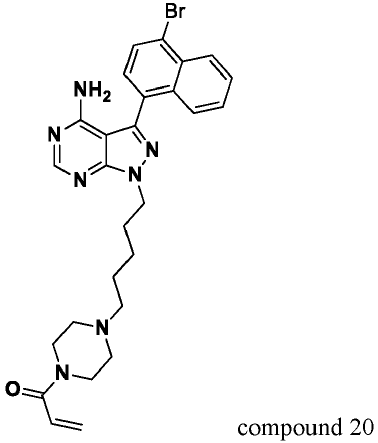

A specific preparation method was the same as the preparation method of compound 1, except that one of the raw materials was equimolar 4-bromonaphthaleneboronic acid, in replacement of 3,5-dimethoxyphenylboronic acid in the preparation method of compound (1-2). NMR test results of the compound 20 were as follows:
¹H NMR (400 MHz, CDCl₃) δ 8.38 (s, 1H), 8.11 (d, *J* = 8.4 Hz, 1H), 7.92 (d, *J* = 8.3 Hz, 1H), 7.64 - 7.42 (m, 4H), 6.53 (dd, *J* = 16.8, 10.5 Hz, 1H), 6.27 (dd, *J* = 16.8, 1.7 Hz, 1H), 5.68 (dd, *J =* 10.5, 1.7 Hz, 1H), 4.51 (t, *J =* 7.1 Hz, 2H), 3.67 (s, 2H), 3.50 (d, *J =* 17.0 Hz, 2H), 2.37 (dd, *J =* 19.3, 12.0 Hz, 6H), 2.13 - 1.99 (m, 2H), 1.58 (dt, *J* = 14.9, 7.6 Hz, 2H), 1.43 (dt, *J* = 15.0, 7.6 Hz, 2H). ¹³C NMR (100 MHz, CDCl₃) δ 165.16, 157.62, 157.55, 155.88, 154.26, 149.40, 134.60, 128.22, 127.80, 127.72, 127.32, 125.04, 123.91, 121.64, 121.60, 110.91, 104.17, 99.87, 97.77, 58.01, 53.21, 52.59, 47.20, 45.47, 41.63, 29.34, 24.31. HRMS (ESI) calculated for C₂₇H₃₀BrN₇NaO+: 570.1587, found 570.1590.

### Example 21: Preparation of compound 21

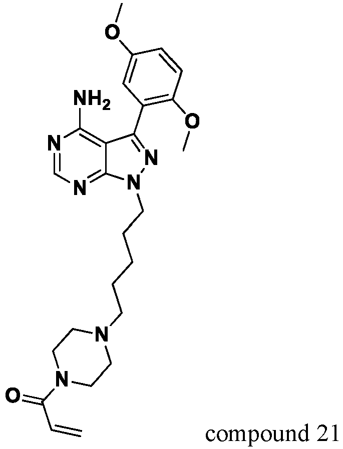

A specific preparation method was the same as the preparation method of compound 1, except that one of the raw materials was equimolar 2,5-dimethoxyphenylboronic acid, in replacement of 3,5-dimethoxyphenylboronic acid in the preparation method of compound (1-2). NMR test results of the compound 21 were as follows:
¹H NMR (400 MHz, CDCl₃) δ 8.35 (s, 1H), 7.07 (s, 1H), 7.02 (d, *J* = 8.6 Hz, 2H), 6.56 (dd, *J =* 16.8, 10.6 Hz, 1H), 6.28 (d, *J =* 16.8 Hz, 1H), 5.70 (d, *J* = 7.6 Hz, 1H), 4.44 (t, *J* = 7.1 Hz, 2H), 3.81 (d, *J* = 11.0 Hz, 6H), 3.69 (s, 2H), 3.55 (s, 2H), 2.47 - 2.32 (m, 6H), 2.03 (dd, *J* = 14.0, 7.0 Hz, 2H), 1.61 - 1.52 (m, 2H), 1.41 (dd, *J* = 14.7, 7.6 Hz, 2H). ¹³C NMR (100 MHz, CDCl₃) δ 165.28, 158.41, 155.55, 154.40, 153.90, 150.49, 140.79, 127.83, 127.47, 123.11, 117.10, 116.10, 113.43, 100.37, 58.17, 56.76, 55.85, 53.35, 52.72, 47.06, 45.66, 41.80, 29.55, 26.21. HRMS (ESI) calculated for C₂₅H₃₃N₇NaO₃⁺: 502.2537, found 502.2539.

### Example 22: Preparation of compound 22

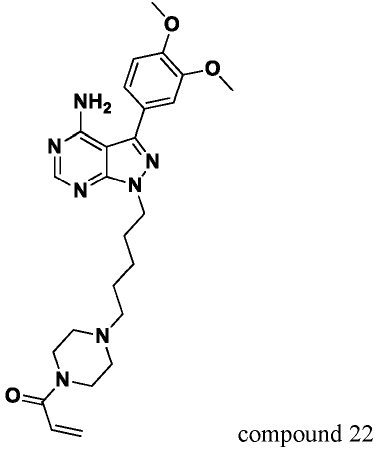

A specific preparation method was the same as the preparation method of compound 1, except that one of the raw materials was equimolar 3,4-dimethoxyphenylboronic acid, in replacement of 3,5-dimethoxyphenylboronic acid in the preparation method of compound (1-2). NMR test results of the compound 22 were as follows:
¹H NMR (400 MHz, CDCl₃) δ 8.39 (s, 1H), 7.27 (s, 1H), 6.89 (s, 2H), 6.64 - 6.50 (m, 1H), 6.29 (d, *J* = 16.6 Hz, 1H), 5.70 (d, *J* = 10.2 Hz, 3H), 4.43 (d, *J* = 6.9 Hz, 2H), 3.94 (s, 3H), 3.91 (s, 3H), 3.69 (s, 2H), 3.56 (s, 2H), 2.38 (dd, *J* = 22.3, 15.1 Hz, 6H), 2.03 - 1.98 (m, 2H), 1.57 (d, *J* = 6.9 Hz, 2H), 1.40 (d, *J* = 7.0 Hz, 2H). ¹³C NMR (100 MHz, CDCl₃)δ 165.34, 165.29, 157.82, 155.94, 155.91, 155.87, 154.22, 154.05, 153.99, 144.18, 138.75, 128.59, 127.92, 127.42, 105.53, 98.33, 61.03, 58.18, 56.33, 53.39, 52.70, 47.06 , 29.61, 26.19, 24.53. HRMS (ESI) calculated for C₂₅H₃₃N₇NaO₃⁺: 502.2537, found 502.2539.

### Example 23: Preparation of compound 23

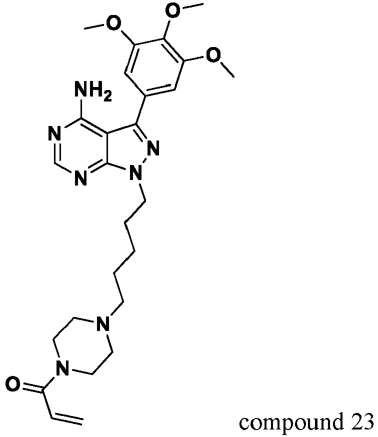

A specific preparation method was the same as the preparation method of compound 1, except that one of the raw materials was equimolar 3,4,5-trimethoxyphenylboronic acid, in replacement of 3,5-dimethoxyphenylboronic acid in the preparation method of compound (1-2). NMR test results of the compound 23 were as follows:
¹H NMR (400 MHz, CDCl₃) *δ* 8.30 (s, 1H), 6.82 (d, *J* = 1.3 Hz, 2H), 6.47 (ddd, *J* = 16.8, 10.5, 1.3 Hz, 1H), 6.19 (dt, *J =* 16.8, 1.6 Hz, 1H), 5.87 (s, 2H), 5.60 (dt, *J* = 10.5, 1.6 Hz, 1H), 4.36 (t, *J =* 7.3 Hz, 2H), 3.86 (d, *J =* 1.3 Hz, 6H), 3.83 (d, *J =* 1.3 Hz, 3H), 3.60 (t, *J =* 5.0 Hz, 2H), 3.47 (t, *J* = 5.0 Hz, 2H), 2.33 (t, *J* = 5.1 Hz, 4H), 2.27 (d, *J* = 7.5 Hz, 2H), 1.93 (q, *J* = 7.6 Hz, 2H), 1.49 (t, *J* = 7.6 Hz, 2H), 1.33 (d, *J* = 7.6 Hz, 2H). ¹³C NMR (100 MHz, CDCl₃) *δ* 165.2, 158.0, 155.8, 154.2, 153.9, 144.1, 138.8, 128.6, 128.6, 128.4, 127.7, 127.5, 105.6, 98.3, 61.0, 58.2, 56.3, 53.4, 52.7, 47.0, 45.7, 41.9, 29.7, 29.6, 26.3, 24.5. HRMS (ESI) calculated for C₂₆H₃₅N₇NaO₄⁺: 532.2643, found 536.2645.

### Example 24: Preparation of compound 24

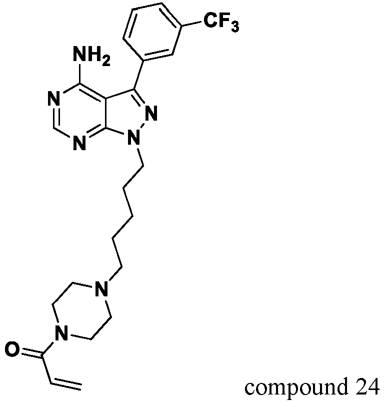

A specific preparation method was the same as the preparation method of compound 1, except that one of the raw materials was equimolar 3-trifluoromethylphenylboronic acid, in replacement of 3,5-dimethoxyphenylboronic acid in the preparation method of compound (1-2). NMR test results of the compound 24 were as follows:
¹H NMR (400 MHz, CDCl₃) *δ* 8.37 (s, 1H), 7.68 - 7.54 (m, 3H), 7.33 (d, *J* = 6.5 Hz, 1H), 6.59 - 6.47 (m, 1H), 6.25 (dd, *J* = 16.8, 4.6 Hz, 1H), 5.95 - 5.59 (m, 3H), 4.43 (d, *J* = 6.6 Hz, 2H), 3.64 (d, *J* = 9.2 Hz, 2H), 3.52 (s, 2H), 2.39 (d, *J* = 5.7 Hz, 4H), 2.32 (d, *J* = 7.3 Hz, 2H), 1.98 (q, *J* = 7.1 Hz, 2H), 1.56 (s, 2H), 1.42 - 1.32 (m, 2H). ¹³C NMR (100 MHz, CDCl₃) *δ* 165.3, 157.7, 155.8, 154.5, 149.8, 142.5, 135.3, 130.9, 127.7, 127.5, 126.7, 121.4, 121.0, 98.3, 58.1, 53.4, 52.7, 47.2, 45.7, 41.9, 29.7, 29.5, 26.2, 24.5. HRMS (ESI) calculated for C₂₄H₂₈F₃N₇NaO⁺: 510.2205, found 510.2207.

### Example 25: Preparation of compound 25

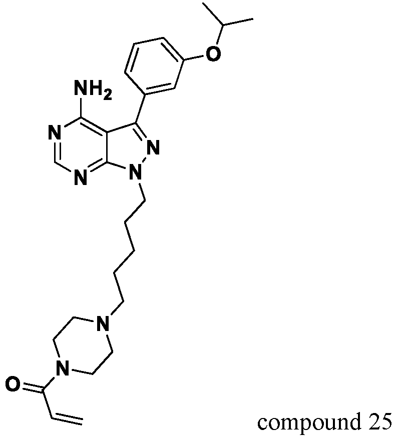

A specific preparation method was the same as the preparation method of compound 1, except that one of the raw materials was equimolar 3-isopropoxyphenylboronic acid, in replacement of 3,5-dimethoxyphenylboronic acid in the preparation method of compound (1-2). NMR test results of the compound 25 were as follows:
¹H NMR (400 MHz, CDCl₃) *δ* 8.15 (d, *J* = 1.2 Hz, 1H), 7.36 (t, *J =* 7.9 Hz, 1H), 7.11 (dd, *J* = 8.2, 5.0 Hz, 2H), 7.00 - 6.95 (m, 1H), 6.70 - 6.60 (m, 1H), 6.09 (dt, *J* = 16.8, 1.6 Hz, 1H), 5.64 (dt, *J =* 10.6, 1.6 Hz, 1H), 4.60 (q, *J* = 6.0 Hz, 1H), 4.32 (t, *J* = 6.8 Hz, 2H), 3.53 (t, *J* = 6.3 Hz, 4H), 2.38 (s, 4H), 2.30 (t, *J* = 7.6 Hz, 2H), 1.87 (d, *J* = 7.1 Hz, 2H), 1.48 (t, *J* = 7.8 Hz, 2H), 1.25 (dd, *J* = 6.0, 1.3 Hz, 6H), 1.22 (d, *J* = 7.4 Hz, 2H). ¹³C NMR (100 MHz, MeOD) *δ* 166.0, 158.6, 158.5, 155.4, 153.7, 144.7, 134.0, 130.2, 127.3, 127.3, 120.1, 116.4, 115.5, 100.0, 97.6, 69.9, 57.6, 52.8, 52.3, 46.4, 45.0, 41.3, 28.9, 25.2, 23.9, 21.0. HRMS (ESI) calculated for C₂₆H₃₅N₇NaO₂⁺: 500.2744, found 500.2747.

### Example 26: Preparation of compound 26

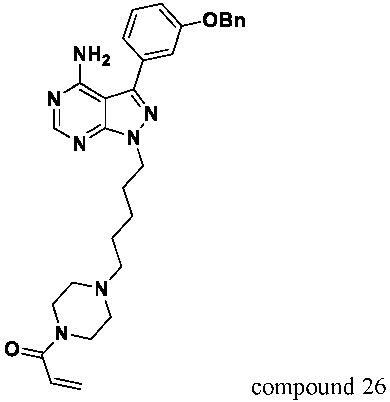

A specific preparation method was the same as the preparation method of compound 1, except that one of the raw materials was equimolar 3-benzyloxyphenylboronic acid, in replacement of 3,5-dimethoxyphenylboronic acid in the preparation method of compound (1-2). NMR test results of the compound 26 were as follows:
¹H NMR (400 MHz, CDCl₃) *δ* 8.37 (s, 1H), 7.49 - 7.27 (m, 8H), 7.11 (d, *J =* 8.5 Hz, 1H), 6.54 (dd, *J =* 16.8, 10.4 Hz, 1H), 6.28 (d, *J =* 16.8 Hz, 1H), 5.69 (d, *J =* 10.4 Hz, 1H), 5.43 (s, 2H), 5.17 (s, 2H), 4.44 (t, *J* = 7.4 Hz, 2H), 3.62 (d, *J* = 49.9 Hz, 4H), 2.39 (d, *J* = 27.3 Hz, 6H), 2.03 - 1.93 (m, 2H), 1.38 (s, 2H). ¹³C NMR (100 MHz, CDCl₃) *δ* 165.3, 159.4, 157.7, 155.4, 154.2, 144.1, 136.6, 134.5, 133.3, 130.6, 128.7, 128.1, 127.7, 127.5, 127.4, 120.9, 116.0, 114.5, 98.2, 70.0, 58.1, 53.3, 52.7, 47.1, 45.7, 41.8, 29.5, 26.2, 24.5, 21.2. HRMS (ESI) calculated for C₃₀H₃₅N₇NaO₂⁺: 548.2744, found 548.2747.

### Example 27: Preparation of compound 27

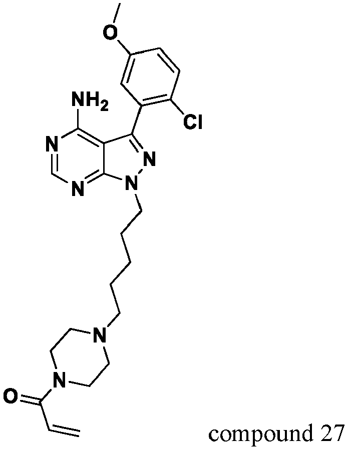

A specific preparation method was the same as the preparation method of compound 1, except that one of the raw materials was equimolar 2-chloro-5-methoxyphenylboronic acid, in replacement of 3,5-dimethoxyphenylboronic acid in the preparation method of compound (1-2). NMR test results of the compound 27 were as follows:
¹H NMR (400 MHz, CDCl₃) *δ* 8.35 (d, *J =* 4.2 Hz, 1H), 7.43 (s, 1H), 6.99 (dq, *J =* 19.8, 3.2 Hz, 2H), 6.53 (ddd, *J* = 15.6, 10.7, 3.9 Hz, 1H), 6.25 (dd, *J* = 16.9, 3.9 Hz, 1H), 5.80 - 5.22 (m, 3H), 4.45 (t, *J* = 6.5 Hz, 2H), 3.82 (d, *J* = 4.9 Hz, 3H), 3.68 - 3.63 (m, 2H), 3.51 (s, 2H), 2.39 (d, *J* = 5.4 Hz, 4H), 2.31 (q, *J* = 6.2, 5.4 Hz, 2H), 1.99 (q, *J* = 6.8 Hz, 2H), 1.53 (q, *J* = 7.1 Hz, 2H), 1.42 - 1.28 (m, 2H). ¹³C NMR (100 MHz, CDCl₃) *δ* 165.3, 158.6, 157.7, 155.9, 153.8, 141.1, 132.6, 131.1, 127.7, 127.5, 124.7, 116.9, 99.9, 70.5, 58.2, 55.7, 53.4, 52.7, 47.1, 45.7, 41.9, 29.5, 26.2, 24.5. HRMS (ESI) calculated for C₂₄H₃₀ClN₇NaO₂⁺: 506.2042, found 506.2044.

### Example 28: Preparation of compound 28

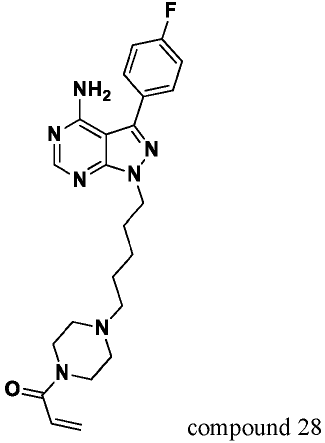

A specific preparation method was the same as the preparation method of compound 1, except that one of the raw materials was equimolar p-fluorophenylboronic acid, in replacement of 3,5-dimethoxyphenylboronic acid in the preparation method of compound (1-2). NMR test results of the compound 28 were as follows:
¹H NMR (400 MHz, CDCl₃) δ 8.31 (s, 1H), 7.25 (s, 2H), 6.54 (dd, *J* = 16.8, 10.5 Hz, 1H), 6.29 (dd, *J* = 16.8, 1.7 Hz, 1H), 6.24 (s, 2H), 5.70 (d, *J* = 10.5 Hz, 1H), 4.35 (t, *J* = 7.0 Hz, 2H), 3.69 (t, *J* = 29.6 Hz, 4H), 2.44 (d, *J* = 31.0 Hz, 6H), 1.92 (dd, *J* = 14.9, 7.4 Hz, 2H), 1.58 (s, 2H), 1.35 - 1.30 (m, 2H). ¹³C NMR (100 MHz, CDCl₃) δ 165.21, 158.54, 157.68, 155.61, 153.68, 141.11, 132.50, 131.04, 127.66, 127.44, 124.68, 116.84, 99.80, 58.07, 55.65, 53.26, 52.63, 47.07, 45.59, 41.74, 29.40, 26.09, 24.40. HRMS (ESI) calculated for C₂₃H₂₈FN₇NaO⁺: 460.2232, found 460.2230.

### Example 29: Preparation of compound 29

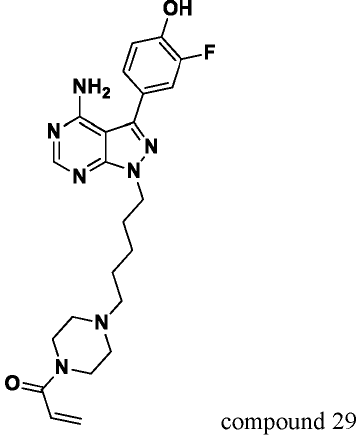

A specific preparation method was the same as the preparation method of compound 1, except that one of the raw materials was equimolar 3-fluoro-4-hydroxyphenylboronic acid, in replacement of 3,5-dimethoxyphenylboronic acid in the preparation method of compound (1-2). NMR test results of the compound 29 were as follows:
¹H NMR (400 MHz, CDCl₃) δ 8.34 (s, 1H), 7.72 - 7.62 (m, 2H), 7.26 - 7.23 (m, 1H), 6.55 (dd, *J* = 16.8, 10.5 Hz, 1H), 6.28 (dd, *J* = 16.8, 1.9 Hz, 1H), 5.69 (dd, *J* = 10.5, 1.9 Hz, 1H), 4.44 (t, *J* = 7.2 Hz, 2H), 3.68 (s, 2H), 3.51 (d, *J =* 21.5 Hz, 2H), 2.47 - 2.39 (m, 4H), 2.36 - 2.31 (m, 2H), 1.99 (dt, *J =* 15.0, 7.5 Hz, 2H), 1.55 (dd, *J* = 15.1, 7.6 Hz, 2H), 1.42 - 1.33 (m, 2H). ¹³C NMR (100 MHz, CDCl₃) δ 165.30, 162.94, 161.55, 157.73, 157.26, 156.54, 154.10, 119.85, 117.25, 100.41, 100.29, 99.99, 61.49, 57.97, 53.20, 52.53, 47.28, 29.69, 29.65, 29.40, 29.26, 24.72, 24.24.. HRMS (ESI) calculated for C₂₃H₂₈FN₇NaO₂+: 476.2181, found 476.2180.

### Example 30: Preparation of compound 30

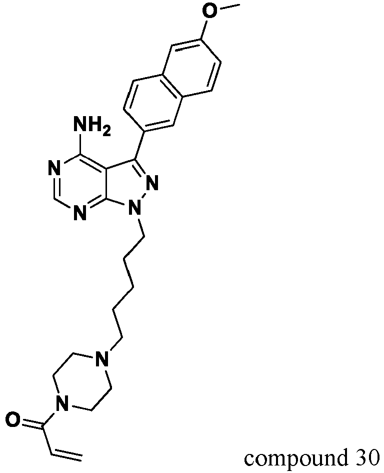

A specific preparation method was the same as the preparation method of compound 1, except that one of the raw materials was equimolar 6-methoxynaphthalene-2-boronic acid, in replacement of 3,5-dimethoxyphenylboronic acid in the preparation method of compound (1-2). NMR test results of the compound 30 were as follows:
¹H NMR (400 MHz, CDCl₃) δ 8.37 (d, *J* = 5.6 Hz, 1H), 8.08 (s, 1H), 7.91 (d, *J* = 8.4 Hz, 1H), 7.83 (d, *J =* 8.9 Hz, 1H), 7.77 (dd, *J =* 8.4, 1.6 Hz, 1H), 7.26 - 7.19 (m, 2H), 6.51 (dd, *J* = 16.8, 10.5 Hz, 1H), 6.31 - 6.16 (m, 1H), 5.77 (d, *J* = 66.8 Hz, 2H), 5.67 (dd, *J =* 10.5, 1.9 Hz, 1H), 4.47 (t, *J =* 7.2 Hz, 2H), 3.97 (s, 3H), 3.69 (s, 2H), 3.54 (s, 2H), 2.38 (dd, *J* = 21.6, 14.2 Hz, 6H), 2.02 (dt, *J* = 14.8, 7.4 Hz, 2H), 1.63 - 1.53 (m, 2H), 1.41 (dt, *J* = 15.1, 7.5 Hz, 2H). ¹³C NMR (100 MHz, CDCl₃) δ 165.27, 158.50, 157.93, 155.54, 154.31, 144.47, 134.66, 129.73, 128.92, 128.23, 128.12, 127.81, 127.54, 127.43, 126.28, 119.94, 105.79, 98.49, 58.13, 55.42, 53.27, 52.70, 47.05, 45.55, 41.71, 29.57, 26.08, 24.51. HRMS (ESI) calculated for C₂₈H₃₃N₇NaO₂⁺: 522.2588, found 522.2587.

### Example 31: Preparation of compound 31

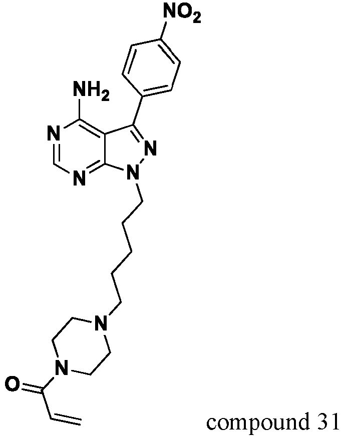

A specific preparation method was the same as the preparation method of compound 1, except that one of the raw materials was equimolar p-nitrophenylboronic acid, in replacement of 3,5-dimethoxyphenylboronic acid in the preparation method of compound (1-2). NMR test results of the compound 31 were as follows:
¹H NMR (400 MHz, CDCl₃) δ 8.45 - 8.36 (m, 3H), 7.92 (d, *J* = 8.8 Hz, 2H), 6.54 (dd, *J* = 16.8, 10.5 Hz, 1H), 6.27 (dd, *J* = 16.9, 2.0 Hz, 1H), 5.90 - 5.63 (m, 3H), 4.48 (t, *J* = 7.2 Hz, 2H), 3.67 (s, 2H), 3.59 - 3.50 (m, 2H), 2.42 (t, *J* = 5.1 Hz, 4H), 2.35 (dd, *J* = 8.5, 6.5 Hz, 2H), 2.01 (p, *J =* 7.6 Hz, 2H), 1.63 - 1.55 (m, 2H), 1.41 (dt, *J =* 11.4, 6.8 Hz, 2H). ¹³C NMR (100 MHz, CDCl₃) *δ* 165.3, 157.6, 155.9, 154.7, 148.0, 141.7, 139.6, 129.2, 127.8, 127.5, 124.6, 98.4, 77.2, 58.1, 53.4, 52.7, 47.4, 45.7, 41.8, 29.7, 29.5, 26.2, 24.5. HRMS (ESI) calculated for C₂₃H₂₈N₈NaO₃⁺: 487.2177, found 487.2179.

### Example 32: Preparation of compound 32

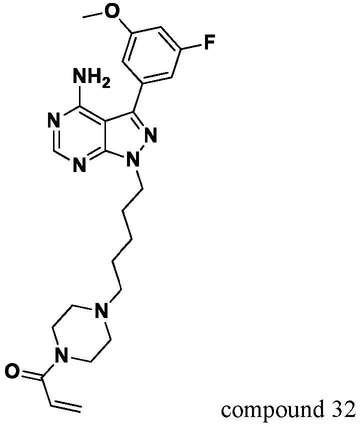

A specific preparation method was the same as the preparation method of compound 1, except that one of the raw materials was equimolar 3-fluoro-5-methoxyphenylboronic acid, in replacement of 3,5-dimethoxyphenylboronic acid in the preparation method of compound (1-2). NMR test results of the compound 31 were as follows:
¹H NMR (400 MHz, CDCl₃) δ 8.38 (s, 1H), 7.24 (d, *J =* 2.1 Hz, 2H), 6.99 - 6.87 (m, 1H), 6.54 (dd, *J =* 16.8, 10.6 Hz, 1H), 6.27 (dd, *J* = 16.8, 1.9 Hz, 1H), 5.80 - 5.63 (m, 3H), 4.44 (t, *J* = 7.2 Hz, 2H), 3.92 (s, 3H), 3.66 (d, *J* = 12.5 Hz, 2H), 3.54 (s, 2H), 2.45 - 2.33 (m, 6H), 2.02 - 1.96 (m, 2H), 1.55 (dd, *J* = 15.0, 7.5 Hz, 2H), 1.37 (dd, *J* = 15.2, 7.2 Hz, 2H). ¹³C NMR (100 MHz, CDCl₃) δ 165.31, 164.85, 164.72, 162.35, 162.23, 157.51, 155.78, 154.50, 127.78, 127.48, 111.59, 111.33, 104.53, 104.28, 98.15, 58.13, 53.38, 52.69, 47.23, 45.66, 41.82, 29.49, 26.17, 24.51. HRMS (ESI) calculated for C₂₄H₃₀FN₇NaO₂⁺: 490.2337, found 490.2335.

### Example 33: Preparation of compound 33

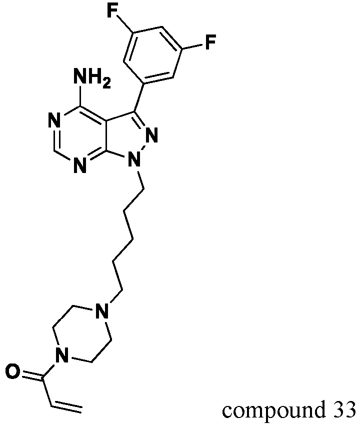

A specific preparation method was the same as the preparation method of compound 1, except that one of the raw materials was equimolar 3,5-difluorophenylboronic acid was used instead of 3,5-dimethoxyphenylboronic acid in the preparation method of compound (1-2). NMR test results of the compound 33 were as follows:
¹H NMR (400 MHz, CDCl₃) δ 8.36 (s, 1H), 7.01 (dd, *J* = 8.4, 2.2 Hz, 2H), 6.73 (dt, *J* = 10.5, 2.3 Hz, 1H), 6.55 (dd, *J* = 16.8, 10.6 Hz, 1H), 6.28 (dd, *J =* 16.8, 1.9 Hz, 1H), 5.83 (s, 2H), 5.69 (dd, *J =* 10.5, 1.9 Hz, 1H), 4.44 (t, *J* = 7.2 Hz, 2H), 3.66 (m, *J* = 16.1 Hz, 2H), 3.55 (m, 2H), 2.37 (dd, *J =* 23.0, 15.7 Hz, 6H), 1.98 (dd, *J* = 14.9, 7.5 Hz, 2H), 1.56 (dd, *J* = 14.8, 7.4 Hz, 2H), 1.45 - 1.36 (m, 2H). ¹³C NMR (100 MHz, CDCl₃) δ 165.28, 162.81, 161.77, 161.65, 157.58, 155.96, 154.41, 127.74, 127.49, 110.07, 110.04, 107.79, 107.57, 102.38, 102.13, 98.25, 58.15, 55.81, 53.35, 52.74, 47.12, 29.51, 24.50. HRMS (ESI) calculated for C₂₃H₂₇F₂N₇NaO⁺: 478.2137, found 478.2139.

### Example 34: Preparation of compound 34

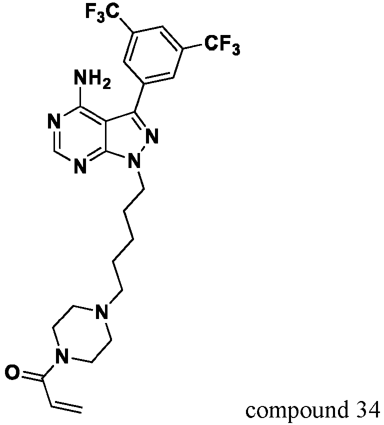

A specific preparation method was the same as the preparation method of compound 1, except that one of the raw materials was equimolar 3,5-bis(trifluoromethyl)phenylboronic acid, in replacement of 3,5-dimethoxyphenylboronic acid in the preparation method of compound (1-2). NMR test results of the compound 34 were as follows:
¹H NMR (400 MHz, CDCl₃) *δ* 8.44 (s, 1H), 8.20 (s, 2H), 7.99 (s, 1H), 6.61 - 6.51 (m, 1H), 6.28 (d, *J =* 17.0 Hz, 1H), 5.69 (d, *J* = 10.6 Hz, 1H), 4.48 (t, *J* = 7.0 Hz, 2H), 3.64 (s, 4H), 2.39 (d, *J* = 26.1 Hz, 6H), 2.05 - 1.99 (m, 2H), 1.78 (s, 2H), 1.38 (dd, *J* = 23.0, 12.9 Hz, 2H). ¹³C NMR (100 MHz, CDCl₃) *δ* 165.3, 157.5, 156.0, 154.8, 140.9, 135.5, 133.2, 132.9, 132.6, 128.5, 127.8, 127.5, 124.4, 122.5, 121.7, 98.4, 58.1, 53.4, 52.7, 47.35 45.7, 41.8, 29.5, 26.2, 24.5. HRMS (ESI) calculated for C₂₅H₂₇F₆N₇NaO⁺: 578.2073, found 578.2075.

### Example 35: Preparation of compound 35

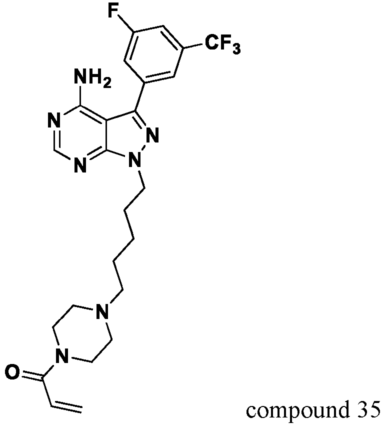

A specific preparation method was the same as the preparation method of compound 1, except that one of the raw materials was equimolar 3-fluoro-5-trifluoromethylphenylboronic acid, in replacement of 3,5-dimethoxyphenylboronic acid in the preparation method of compound (1-2). NMR test results of the compound 35 were as follows:
¹H NMR (400 MHz, CDCl₃) *δ* 8.35 (s, 1H), 7.73 (s, 1H), 7.58 (dd, *J* = 8.7, 2.0 Hz, 1H), 7.40 - 7.37 (m, 1H), 6.48 (dd, *J* = 16.8, 10.5 Hz, 1H), 6.20 (dd, *J* = 16.8, 1.9 Hz, 1H), 5.61 (dd, *J* = 10.5, 1.9 Hz, 1H), 4.39 (t, *J* = 7.2 Hz, 2H), 3.60 (s, 2H), 3.47 (s, 2H), 2.34 (t, *J* = 5.1 Hz, 4H), 2.30 - 2.24 (m, 2H), 1.96 - 1.90 (m, 2H), 1.52 - 1.47 (m, 2H), 1.32 (t, *J* = 7.6 Hz, 2H). ¹³C NMR (100 MHz, CDCl₃) *δ* 162.0, 161.5, 157.8, 155.6, 154.1, 144.2, 135.0, 129.5, 127.8, 127.7, 106.4, 101.0, 98.2, 58.0, 55.6, 53.3, 52.5, 47.0, 46.0, 42.2, 29.7, 29.5, 26.2, 24.5. HRMS (ESI) calculated for C₂₄H₂₇F₄N₇NaO⁺: 528.2105, found 528.2107.

### Example 36: Preparation of compound 36

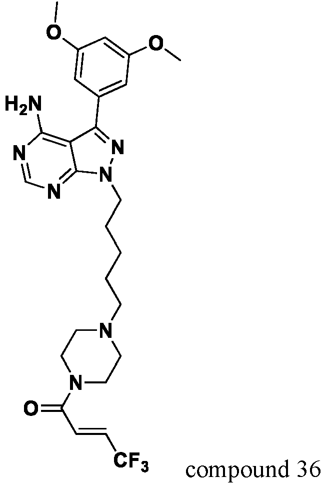

A specific preparation method was the same as the preparation method of compound 10, except that one of the raw materials was equimolar 4,4,4-trifluorocrotonic acid, in replacement of acryloyl chloride in the preparation method of compound (1-3). NMR test results of the compound 36 were as follows:
¹H NMR (400 MHz, CDCl₃) *δ* 8.32 (s, 1H), 6.92 (dt, *J* = 15.4, 2.1 Hz, 1H), 6.78 (d, *J*= 2.3 Hz, 2H), 6.73 - 6.64 (m, 1H), 6.52 (t, *J* = 2.3 Hz, 1H), 4.40 (t, *J* = 7.2 Hz, 2H), 3.82 (s, 6H), 3.65 (t, *J* = 5.2 Hz, 2H), 3.50 (t, *J* = 5.0 Hz, 2H), 2.40 (q, *J* = 4.7 Hz, 4H), 2.32 (t, *J =* 7.5 Hz, 2H), 1.96 (p, *J* = 7.4 Hz, 2H), 1.52 (q, *J* = 7.6 Hz, 2H), 1.36 (q, *J* = 8.2 Hz, 2H). ¹³C NMR (100 MHz, CDCl₃) *δ* 162.3, 161.8, 158.1, 155.8, 154.3, 144.4, 135.3, 129.7, 129.4, 128.0, 127.9, 121.4, 106.6, 101.3, 98.5, 58.3, 55.8, 53.5, 52.7, 47.3, 46.2, 42.5, 29.9, 29.8, 26.4, 24.7. HRMS (ESI) calculated for C₂₆H₃₂F₃N₇NaO₃⁺: 570.2411, found 570.2413.

### Example 37: Preparation of compound 37

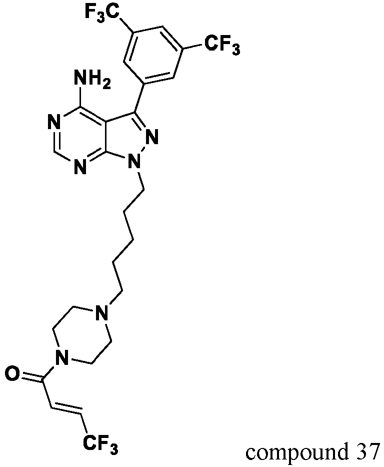

A specific preparation method was the same as the preparation method of compound 34, except that one of the raw materials was equimolar 4,4,4-trifluorobutenoic acid in replacement of acryloyl chloride in the preparation method of compound (1-3). NMR test results of the compound 37 were as follows:
¹H NMR (400 MHz, CDCl₃) *δ* 8.43 (d, *J* = 2.3 Hz, 1H), 8.20 (s, 2H), 7.99 (s, 1H), 6.94 (d, *J* = 15.5 Hz, 1H), 6.72 (dt, *J* = 14.9, 6.8 Hz, 1H), 4.48 (t, *J* = 7.0 Hz, 2H), 3.69 (s, 2H), 3.54 (s, 2H), 2.41 (d, *J* = 31.2 Hz, 6H), 2.02 (q, *J* = 7.8 Hz, 2H), 1.59 (s, 2H), 1.44 - 1.26 (m, 2H). ¹³C NMR (100 MHz, CDCl₃) *δ* 164.1, 162.1, 161.6, 157.4, 155.9, 154.7, 141.3, 136.6, 127.7, 121.0, 118.9, 118.7, 113.4, 113.2, 98.2, 58.0, 53.3, 52.5, 47.2, 45.9, 42.2, 29.7, 29.5, 26.1, 24.5, 14.1. HRMS (ESI) calculated for C₂₆H₂₆F₉N₇NaO⁺: 646.1947, found 646.1947.

### Example 38: Preparation of Compound 38

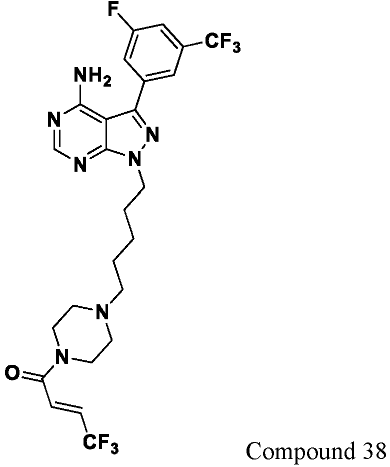

A specific preparation method was the same as the preparation method of compound 35, except that one of the raw materials was equimolar 4,4,4-trifluorobutenoic acid, in replacement of acryloyl chloride in the preparation method of compound (1-3). NMR test results of the compound 38 were as follows:
¹H NMR (400 MHz, CDCl₃) *δ* 8.43 (d, *J* = 2.3 Hz, 1H), 8.20 (s, 2H), 7.99 (s, 1H), 6.94 (d, *J* = 15.5 Hz, 1H), 6.71 (dq, *J* = 14.2, 6.7 Hz, 1H), 4.48 (t, *J* = 7.0 Hz, 2H), 3.69 (s, 2H), 3.54 (s, 2H), 2.41 (d, *J* = 31.2 Hz, 6H), 2.01 (t, *J* = 7.6 Hz, 2H), 1.59 (s, 2H), 1.48 - 1.27 (m, 2H). ¹³C NMR (100 MHz, CDCl₃) *δ* 162.0, 155.2, 154.6, 142.9, 133.2, 132.1, 132.1, 129.4, 129.0, 128.6, 128.5, 127.9, 121.3, 120.8, 111.6, 105.6, 58.1, 53.3, 52.6, 46.9, 46.0, 42.3, 29.5, 26.3, 24.5, 22.1. HRMS (ESI) calculated for C₂₆H₂₆F₉N₇NaO⁺: 646.1947, found 646.1947.

### Example 39: Preparation of compound 39

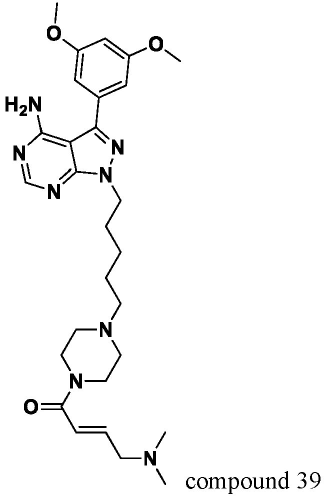

A specific preparation method was the same as the preparation method of compound 10, except that one of the raw materials was equimolar trans-4-dimethylaminocrotonic acid, in replacement of acryloyl chloride in the preparation method of compound (1-3). NMR test results of the compound 39 were as follows:
¹H NMR (400 MHz, CDCl₃) *δ* 8.38 (s, 1H), 6.81 (d, *J* = 2.3 Hz, 2H), 6.56 (t, *J* = 2.3 Hz, 1H), 5.60 (s, 2H), 4.43 (t, *J* = 7.2 Hz, 2H), 3.86 (s, 6H), 3.67 (t, *J =* 5.1 Hz, 2H), 3.59 (t, *J =* 5.1 Hz, 2H), 3.49 (s, 1H), 2.45 (t, *J* = 5.1 Hz, 4H), 2.33 (t, *J* = 7.4 Hz, 2H), 1.96 (d, *J* = 7.5 Hz, 2H), 1.50 - 1.35 (m, 4H). ¹³C NMR (100 MHz, CDCl₃) *δ* 161.5, 161.5, 157.7, 155.9, 144.0, 135.1, 106.4, 101.0, 100.0 98.3, 58.1, 55.6, 53.1, 52.4, 52.3, 49.4, 47.1, 45.8, 43.4, 43.3, 43.2, 37.4, 29.6, 26.9, 26.5. HRMS (ESI) calculated for C₂₅H₃₁N₇NaO₃⁺: 500.2831, found 500.2833.

### Example 40: Preparation of compound 40

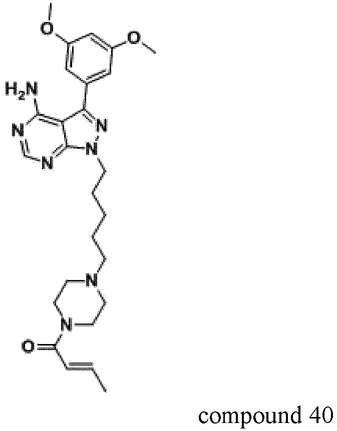

A specific preparation method was the same as the preparation method of compound 10, except that one of the raw materials was equimolar crotonic acid, in replacement of acryloyl chloride in the preparation method of compound (1-3). NMR test results of the compound 40 were as follows:
¹H NMR (400 MHz, CDCl₃) *δ* 8.31 (s, 1H), 6.88 - 6.73 (m, 3H), 6.52 (t, *J* = 2.3 Hz, 1H), 6.20 (dq, *J* = 15.0, 1.7 Hz, 1H), 4.39 (t, *J* = 7.2 Hz, 2H), 3.82 (s, 6H), 3.62 (d, *J* = 8.1 Hz, 2H), 3.56 - 3.43 (m, 2H), 2.36 (t, *J =* 5.1 Hz, 4H), 2.29 (dd, *J* = 8.7, 6.4 Hz, 2H), 2.01 - 1.91 (m, 2H), 1.83 (dd, *J* = 6.9, 1.7 Hz, 3H), 1.52 (t, *J* = 7.6 Hz, 2H), 1.36 (ddd, *J* = 15.3, 9.1, 5.4 Hz, 2H). ¹³C NMR (100 MHz, CDCl₃) *δ* 165.5, 161.5, 158.0, 155.7, 154.1, 144.1, 141.5, 135.1, 121.4, 119.9, 106.4, 101.1, 98.2, 60.4, 58.2, 55.5, 53.5, 53.4, 52.8, 47.0, 45.5, 41.8, 29.6, 26.2, 24.5, 18.2. HRMS (ESI) calculated for C₂₆H₃₅N₇NaO₃⁺: 516.2694, found 516.2696.

### Example 41 Enzyme activity test

In the present disclosure, the homogeneous time-resolved fluorescence (HTRF) technology was used to evaluate an inhibitory effect of a compound on the four subtypes FGFR1, FGFR2, FGFR3, and FGFR4. FGFR1, FGFR2, FGFR3, and FGFR4 prokaryotic expression vectors were first constructed; and when the prokaryotic expression vectors were identified as correct, FGFR proteins were expressed and purified on a large scale in an *Escherichia coli* (*E. coli*) system, and protein concentrations were determined. Resulting proteins were stored in aliquots. Before an enzyme activity test was started, cisbio HTRF KinEA (62TK0PEB) was used to predilute each component required in an enzyme activity system with an enzyme activity buffer to a required working concentration. A 384-well plate (66PL384025) was prepared, 5 µL of a protein solution, 1 µL of a compound, and 2 µL of a substrate were added to each well, and 2 µL of ATP was finally added to initiate a reaction. Three parallel experiments were set for each concentration. In the blank control group, 1 µL of the compound was replaced with 1 µL of dimethyl sulfoxide (DMSO); and in the positive control group, the positive drug AZD4547 was used instead, and the other conditions remained unchanged. The 384-well plate was sealed and incubated at room temperature for about 1 h. After the incubation was completed, 5 µL of a lanthanide-labeled phosphorylated substrate antibody (a donor) and 5 µL of a streptavidin-labeled XL665 (a receptor) were added to each well to stop the reaction, and the plate was further incubated at room temperature for about 1 h in the dark. Finally, the TECAN infinite M1000PRO multi-function microplate reader was used to detect a fluorescence signal, an inhibition rate was calculated, and an IC50 value was fitted with GraphPad Prism 7.0.

Results showed that the compounds 1 to 40 could inhibit the protein activity of FGFR1/2/3/4 to varying degrees.

**Table 1 Structure-activity relationship (SAR) studies based on enzyme activity results**

| 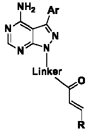 | | | | | | | |
|---|---|---|---|---|---|---|---|
| Comp ound No. | Ar | Linker | R | Enzyme activity (IC50/nM) | | | |
| | | | | FGFR1 | FGFR2 | FGFR3 | FGFR4 |
| 1 | | 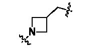 | | 315 | 540 | 520 | 1921 |
| 2 | | 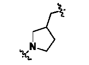 | | 818 | 666 | 525 | >5000 |
| 3 | | 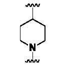 | | 433 | 424 | 371 | 2730 |
| 4 | 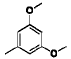 | 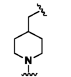 | H | 615 | 641 | 521 | >5000 |
| 5 | | 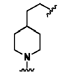 | | 741 | 1210 | 580 | 1780 |
| 6 | | 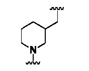 | | 790 | 1130 | 769 | >5000 |
| 7 | | 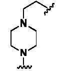 | | 372 | 455 | 335 | 1610 |
| 8 | | 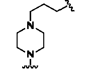 | | 323 | 351 | 414 | 4700 |
| 9 | | 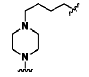 | | 36.0 | 27.4 | 22.5 | 4210 |
| 10 | | 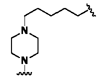 | | 3.2 | 1.4 | 2.7 | 3270 |
| 11 | | 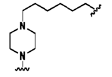 | | 12.9 | 11.2 | 11.3 | 3207 |
| 12 | | 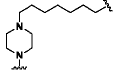 | | 5.9 | 2.2 | 3.4 | >5000 |
| 13 | 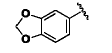 | | | 476 | 789 | 875 | 2709 |
| 14 | 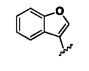 | | | 2109 | 1864 | 344 | >5000 |
| 15 | 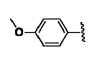 | | | >5000 | 3121 | >5000 | >5000 |
| 16 | 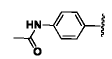 | | | 1353 | 2511 | 1321 | >5000 |
| 17 | 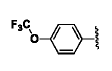 | | | 3523 | 4111 | >5000 | >5000 |
| 18 | 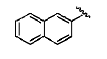 | 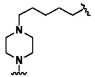 | | >5000 | >5000 | >5000 | >5000 |
| 19 | 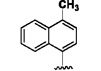 | | | >5000 | >5000 | >5000 | >5000 |
| 20 | 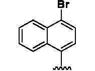 | | | >5000 | >5000 | >5000 | >5000 |
| 21 | 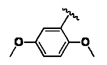 | | | 866 | 954 | 1978 | >5000 |
| 22 | 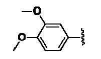 | | | 324 | 231 | 523 | >5000 |
| 23 | 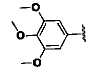 | | | 277 | 223 | 486 | 4910 |
| 24 | 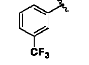 | | | 1877 | >5000 | >5000 | 3903 |
| 25 | 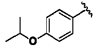 | | | >5000 | >5000 | >5000 | 2246 |
| 26 | 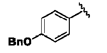 | | | 1501 | 2345 | >5000 | >5000 |
| 27 | 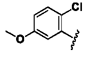 | | | 554 | 763 | 731 | 2723 |
| 28 | 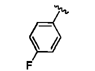 | | | 185 | 337 | 321 | 2987 |
| 29 | 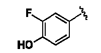 | | | 417 | 560 | 318 | 2580 |
| 30 | 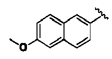 | | | >5000 | >5000 | >5000 | >5000 |
| 31 | 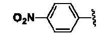 | | | >5000 | >5000 | >5000 | >5000 |
| 32 | 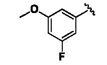 | | | 118 | 133 | 132 | >5000 |
| 33 | 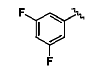 | | | 790 | >10 | >10 | 4112 |
| 34 | 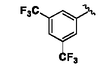 | | | 133 | 165 | 115 | 3921 |
| 35 | 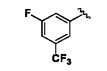 | | | 14.1 | 29.3 | 13.2 | 4102 |
| 36 | 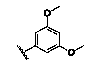 | | CF₃ | 0.7 | 0.7 | 0.9 | 809 |
| 37 | 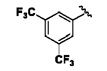 | | | 9.9 | 12.1 | 10.2 | >5000 |
| 38 | 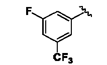 | | | 101 | 65 | 97 | 2401 |
| 39 | 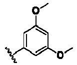 | | (CH₃)₂NCH₂ | 13.8 | 37.5 | 15.5 | >5000 |
| 40 | | | CH₃ | 16.6 | 26.3 | 12.4 | >5000 |
| AZD4 547 | NA | | | 1.1 | 2.2 | 1.8 | 660 |

### Example 42 Cell proliferation assay

In the present disclosure, the compounds 10 and 36 were selected, and the methyl thiazolyl tetrazolium (MTT) colorimetric method was used to evaluate the proliferation inhibition effects of the compounds 10 and 36 on various tumor cells. Tumor cells in a logarithmic growth phase were collected through trypsinization and resuspended in 1 mL of a fresh medium, and a small amount of a resulting suspension was taken, diluted, and subjected to cell counting with a hemocytometer. According to the cell growth rate, tumor cells were inoculated into a 96-well plate at a density of 3,000 cells/well to 5,000 cells/well, and then cultivated in an incubator for 24 h. The tumor cells were treated with the compounds 10 and 36 at 8 concentrations, with three replicate wells for each concentration, and then incubated for 96 h. After the incubation was completed, 10 µL of an MTT solution (5 mg/mL) was added to each well, and the plate was tapped for thorough mixing and then further incubated in an incubator for 3 h to 4 h. After the incubation was completed, the liquid in the well plate was carefully pipetted and removed, and then 100 µL of a DMSO solution was added to each well. Programs of a microplate reader were set to determine the absorbance values at 490 nm and 570 nm respectively, a proliferation inhibition rate at each concentration was calculated according to a formula, and the Graphpad Prism 7 software was finally used for data processing to calculate IC50.

The results (Table 1) showed that the compounds 10 and 36 significantly inhibited the proliferation of human squamous cell lung carcinoma cell NCI-H1581 (FGFR1 amplification) and human gastric cancer cell SNU-16 (FGFR2 amplification) and inhibited the proliferation of human hepatoma cell SK-hep-1 (FGFR4 amplification) to some degree, but exhibited no obvious inhibitory effect on tumor cells with normal FGFR expression, indicating that the compounds 10 and 36 had strong selectivity.

**Table 2 Anti-proliferation activities of the compound 36 for specific cancer cell lines**

| Compound | IC50/µM | | | | |
|---|---|---|---|---|---|
| | NCI-H1581 FGFR1 amplification | SNU-16 FGFR2 amplification | SK-hep-1 FGFR4 amplification | Huh-7 | A549 |
| AZD4547 | 0.042±0.002 | 0.006±0.001 | 2.78±0.069 | >10 | >10 |
| 10 | 0.051±0.003 | 0.037±0.005 | 2.86±0.250 | >10 | >10 |
| 36 | 0.055±0.008 | 0.043±0.006 | 3.05±0.120 | >10 | >10 |

### Example 43 Inhibition of the compounds 10 and 36 on FGF/FGFR and downstream signaling pathways thereof

To further elucidate the influence of the compounds 10 and 36 on FGF/FGFR signaling, the influence of the compounds on FGFR and downstream signaling protein phosphorylation was verified by western blot (WB). Each of human squamous cell lung carcinoma cells NCI-H1581 and human gastric cancer cells SNU-16 in a logarithmic growth phase were collected through trypsinization and resuspended in 1 mL of a fresh medium, and a small volume of a resulting tumor cell suspension was taken, diluted, and subjected to cell counting with a hemocytometer. A corresponding volume of the tumor cell suspension was taken and inoculated into a 6-well plate at a density of 3 × 10⁵ cells/well, and cultivated in an incubator for 24 h. After the cells were completely adherence, the used medium was pipetted and removed, fresh media with the compounds 10 and 36 at different concentrations were added, and the plate was incubated in an incubator for 12 h. After the incubation was completed, a culture supernatant was pipetted and removed, and the plate was washed three times with a pre-cooled 1X PBS solution to completely pipet and remove the residual solution; 60 µL of an RIPA lysis buffer was added to each well, and cells were gently scraped off with a cell scraper; a resulting lysis buffer was transferred to a 1.5 mL EP tube, subjected an ice bath for 10 min, and centrifuged at 14,000 rpm for 10 min, a resulting supernatant was pipetted into a new 1.5 mL EP tube, and a small amount of the supernatant was pipetted for protein content determination; and a corresponding volume of 5 X loading buffer was added to the remaining supernatant, and a resulting mixture was vortexed for thorough mixing and subjected a metal bath at 100°C for 10 min. According to a determined protein content, a corresponding loading amount was calculated at 30 µg of protein per well. Proteins were isolated by 8% sodium dodecyl sulfate-polyacrylamide gel electrophoresis (SDS-PAGE), the isolated proteins were transferred to a polyvinylidene fluoride (PVDF) membrane, and stained with Ponceau S, and the PDVF membrane with proteins was cut according to a molecular weight of a target protein, rinsed with a 1X TBST solution to remove Ponceau S, and blocked with 5% skimmed milk powder for 30 min. The PDVF membrane was washed three times with a 1X TBST solution, a PDVF membrane with the target protein band was placed in an incubation box, a corresponding primary antibody incubation solution was added, and the PDVF membrane was incubated at 4°C overnight. After the primary antibody incubation was completed, the membrane was washed three times with a 1X TBST solution, a corresponding secondary antibody incubation solution was added, and the membrane was incubated at room temperature for 1.5 h. After the incubation was completed, the membrane was washed three times with a 1X TBST solution and subjected to color development with a hypersensitive horseradish peroxidase (HRP) chemiluminescence kit.

As shown in FIG. 1 and FIG. 2, the compounds 10 and 36 not only significantly inhibited the phosphorylation of FGFR1 in NCI-H1581 cells and the phosphorylation of FGFR2 in SNU-16 cells, but also inhibited the activation of downstream PLCγ, AKT, and ERK in a dose-dependent manner. In a word, the compounds 10 and 36 can strongly inhibit FGF/FGFR and downstream signaling pathways *in vitro.*

### Example 44 Significant inhibition of the compounds 10 and 36 on tumor growth in mice

In order to evaluate an anti-tumor effect of the compounds 10 and 36 *in vivo,* a mouse subcutaneous xenograft model was established with human squamous cell lung carcinoma cells NCI-H1581 with high FGFR1 expression. Cells in a logarithmic growth phase were collected through trypsinization, washed three times with 1X PBS. A resulting cell precipitation was diluted to 1 × 10⁸ cells/mL with a resuspending solution (1640 medium : Matrigel = 1:1), and inoculated into inferior extremity of armpits of anterior limbs of 5-6 week-old female BALB/c Nude mice at 5 × 10⁶ cells/mouse. When a tumor volume grew to 50 mm³ to 100 mm³, the mice were randomly divided into three groups (control group, 50 mg/kg, and 100 mg/kg), with 6 mice in each group. The compound 10 was administered orally every day, and the compound 36 was intraperitoneally injected every day. The tumor volume and mouse body weight were measured. The mice was treated after 26 d of administration, that a tumor tissue was dissected and fixed in a formalin solution for later use.

As shown in FIG. 3 and FIG. 4, the compounds 10 and 36 inhibited the proliferation of NCI-H1581 cells in mice in a dose-dependent manner, with an inhibition rate of 72% in the highdose group; inhibited the phosphorylation activation of FGFR1 in a tumor tissue; and did not lead to an obvious weight loss, indicating that the compounds 10 and 36 were well tolerated *in vivo* at all doses.

In conclusion, the present disclosure optimizes 2H-pyrazolo[3,4-d]pyrimidine to obtain novel derivatives thereof (compounds 10 and 36), and the compounds 10 and 36 are served as effective and selective inhibitors for FGFR1, FGFR2, and FGFR3, which exhibit an irreversible binding effect to a target protein. The compounds are leading compounds that can not only inhibit FGF/FGFR and downstream signaling pathways thereof at low concentrations, but also exhibit significant anti-proliferation effects on the NCI-H1581 cancer cell line *in vitro* and *in vivo.* In addition, the compounds 10 and 36 show low toxicity and prominent PK properties, and are currently identified as potential drug candidates.

The above are merely examples of the present disclosure, and are not intended to limit the present disclosure.

### References

(1) Turner, N.; Grose, R. Fibroblast growth factor signalling: from development to cancer. Nat. Rev. Cancer 2010, 10, 116-129.
(2) Carter, E. P.; Fearon, A. E.; Grose, R. P. Careless talk costs lives: fibroblast growth factor receptor signalling and the consequences of pathway malfunction. Trends Cell Biol. 2015, 25, 221-233.
(3) Touat, M.; Ileana, E.; Postel-Vinay, S.; Andre, F.; Soria, J. C. Targeting FGFR signaling in cancer. Clin. Cancer Res. 2015, 21, 2684-2694.
(4) Dieci, M. V.; Arnedos, M.; Andre, F.; Soria, J. C. Fibroblast growth factor receptor inhibitors as a cancer treatment: from a biologic rationale to medical perspectives. Cancer Discovery 2013, 3, 264-279.
(5) Hagel, M.; Miduturu, C.; Sheets, M.; Rubin, N.; Weng, W.; Stransky, N.; Bifulco, N.; Kim, J. L.; Hodous, B.; Brooijmans, N.; Shutes, A.; Winter, C.; Lengauer, C.; Kohl, N. E.; Guzi, T. First selective small molecule inhibitor of FGFR4 for the treatment of hepatocellular carcinomas with an activated FGFR4 signaling pathway. Cancer Discovery 2015, 5, 424-437.
(6) Babina, I. S.; Turner, N. C. Advances and challenges in targeting FGFR signalling in cancer. Nat. Rev. Cancer 2017, 17, 318-332.
(7) Renhowe, P. A.; Pecchi, S.; Shafer, C. M.; Machajewski, T. D.; Jazan, E. M.; Taylor, C.; Antonios-McCrea, W.; McBride, C. M.; Frazier, K.; Wiesmann, M.; Lapointe, G. R.; Feucht, P. H.; Warne, R. L.; Heise, C. C.; Menezes, D.; Aardalen, K.; Ye, H.; He, M.; Le, V.; Vora, J.; Jansen, J. M.; Wernette-Hammond, M. E.; Harris, A. L. Design, structure-activity relationships and in vivo characterization of 4-amino-3-benzimidazol-2-ylhydroquinolin-2-ones: a novel class of receptor tyrosine kinase inhibitors. J. Med. Chem. 2009, 52, 278-292.
(8) Bello, E.; Colella, G.; Scarlato, V.; Oliva, P.; Berndt, A.; Valbusa, G.; Serra, S. C.; D'Incalci, M.; Cavalletti, E.; Giavazzi, R.; Damia, G.; Camboni, G. E-3810 is a potent dual inhibitor of VEGFR and FGFR that exerts antitumor activity in multiple preclinical models. Cancer Res. 2011, 71, 1396-1405.
(9) Roth, G. J.; Heckel, A.; Colbatzky, F.; Handschuh, S.; Kley, J.; Lehmann-Lintz, T.; Lotz, R.; Tontsch-Grunt, U.; Walter, R.; Hilberg, F. Design, synthesis, and evaluation of indolinones as triple angiokinase inhibitors and the discovery of a highly specific 6-methoxycarbonyl-substituted indolinone (BIBF 1120). J. Med. Chem. 2009, 52, 4466-4480.
(10) Gozgit, J. M.; Wong, M. J.; Moran, L.; Wardwell, S.; Mohemmad, Q. K.; Narasimhan, N. I.; Shakespeare, W. C.; Wang, F.; Clackson, T.; Rivera, V. M. Ponatinib (AP24534), a multitargeted pan-FGFR inhibitor with activity in multiple FGFR-amplified or mutated cancer models. Mol. Cancer Ther. 2012, 11, 690-699.
(11) Gavine, P. R.; Mooney, L.; Kilgour, E.; Thomas, A. P.; Al-Kadhimi, K.; Beck, S.; Rooney, C.; Coleman, T.; Baker, D.; Mellor, M. J.; Brooks, A. N.; Klinowska, T. AZD4547: an orally bioavailable, potent, and selective inhibitor of the fibroblast growth factor receptor tyrosine kinase family. Cancer Res. 2012, 72, 2045-2056.
(12) Guagnano, V.; Furet, P.; Spanka, C.; Bordas, V.; Le Douget, M.; Stamm, C.; Brueggen, J.; Jensen, M. R.; Schnell, C.; Schmid, H.; Wartmann, M.; Berghausen, J.; Drueckes, P.; Zimmerlin, A.; Bussiere, D.; Murray, J.; Graus Porta, D. Graus Porta, D. Discovery of 3-(2,6-dichloro-3,5-dimethoxy-phenyl)-1-{6-[4-(4-ethyl-piperazin-1-yl)-phenylamino]-pyrimidin-4-yl}-1-methyl-urea (NVP-BGJ398), a potent and selective inhibitor of the fibroblast growth factor receptor family of receptor tyrosine kinase. J. Med. Chem. 2011, 54, 7066-7083.
(13) Zhao, G.; Li, W. Y.; Chen, D.; Henry, J. R.; Li, H. Y.; Chen, Z.; Zia-Ebrahimi, M.; Bloem, L.; Zhai, Y.; Huss, K.; Peng, S. B.; McCann, D. J. A novel, selective inhibitor of fibroblast growth factor receptors that shows a potent broad spectrum of antitumor activity in several tumor xenograft models. Mol. Cancer Ther. 2011, 10, 2200-2210.
(14) Perera, T. P. S.; Jovcheva, E.; Mevellec, L.; Vialard, J.; De Lange, D.; Verhulst, T.; Paulussen, C.; Van De Ven, K.; King, P.; Freyne, E.; Rees, D. C.; Squires, M.; Saxty, G.; Page, M.; Murray, C. W.; Gilissen, R.; Ward, G.; Thompson, N. T.; Newell, D. R.; Cheng, N.; Xie, L.; Yang, J.; Platero, S. J.; Karkera, J. D.; Moy, C.; Angibaud, P.; Laquerre, S.; Lorenzi, M. V. Discovery and pharmacological characterization of JNJ-42756493 (erdafitinib), a functionally selective smallmolecule FGFR family inhibitor. Mol. Cancer Ther. 2017, 16, 1010-1020.
(15) Zhou, W.; Hur, W.; McDermott, U.; Dutt, A.; Xian, W.; Ficarro, S. B.; Zhang, J.; Sharma, S. V; Brugge, J.; Meyerson, M.; Settleman, J.; Gray, N. S. A structure-guided approach to creating covalent FGFR inhibitors. Chem. Biol. 2010, 17, 285-295.
(16) Tan, L.; Wang, J.; Tanizaki, J.; Huang, Z.; Aref, A. R.; Rusan, M.; Zhu, S. J.; Zhang, Y.; Ercan, D.; Liao, R. G.; Capelletti, M.; Zhou, W.; Hur, W.; Kim, N.; Sim, T.; Gaudet, S.; Barbie, D. A.; Yeh, J. R. J.; Yun, C. H.; Hammerman, P. S.; Mohammadi, M.; Janne, P. A.; Gray, N. S. Development of covalent inhibitors that can overcome resistance to first-generation FGFR kinase inhibitors. Proc. Natl. Acad. Sci. U. S. A. 2014, 111, E4869-E4877.
(17) Brown, W. S.; Tan, L.; Smith, A.; Gray, N. S.; Wendt, M. K. Covalent targeting of fibroblast growth factor receptor inhibits metastatic breast cancer. Mol. Cancer Ther. 2016, 15, 2096-2106.
(18) Brameld, K. A.; Owens, T. D.; Verner, E.; Venetsanakos, E.; Bradshaw, J. M.; Phan, V. T.; Tam, D.; Leung, K.; Shu, J.; LaStant, J.; Loughhead, D. G.; Ton, T.; Karr, D. E.; Gerritsen, M. E.; Goldstein, D. M.; Funk, J. O. Discovery of the irreversible covalent FGFR inhibitor 8-(3-(4-acryloylpiperazin-1-yl)propyl)-6-(2,6-dichloro-3,5-dimethoxyphenyl)-2-(methylamino)pyrido[2,3-d]pyrimidin-7(8H)-one (PRN1371) for the treatment of solid tumors. J. Med. Chem. 2017, 60, 6516-6527.
(19) Wang, Y.; Dai, Y.; Wu, X.; Li, F.; Liu, B.; Li, C.; Liu, Q.; Zhou, Y; Wang, B.; Zhu, M.; Cui, R.; Tan, X.; Xiong, Z.; Liu, J.; Tan, M.; Xu, Y.; Geng, M.; Jiang, H.; Liu, H.; Ai, J.; Zheng, M. Discovery and Development of a Series of Pyrazolo[3,4-d]pyridazinone Compounds as the Novel Covalent Fibroblast Growth Factor Receptor Inhibitors by the Rational Drug Design. J. Med. Chem. 2019, 62, 7473-7488.
(20) Wang, Y.; Li, L.; Fan, J.; Dai, Y.; Jiang, A.; Geng, M.; Ai, J.; Duan, W. Discovery of Potent Irreversible Pan-Fibroblast Growth Factor Receptor (FGFR) Inhibitors. J. Med. Chem. 2018, 61, 9085-9104.
(21) Li, X.; Guise, C. P.; Taghipouran, R.; Yosaatmadja, Y.; Ashoorzadeh, A.; Paik, W.; Squire, C. J.; Jiang, S.; Luo, J.; Xu, Y.; Tu, Z.; Lu, X.; Ren, X.; Patterson, A. V.; Smaill, J. B.; Ding, K. 2-Oxo-3, 4-dihydropyrimido[4, 5-d]pyrimidinyl derivatives as new irreversible pan fibroblast growth factor receptor (FGFR) inhibitors. Eur. J. Med. Chem. 135, 2017, 531-543.
(22) AOLI WANG et al, "Discovery of (R)-1-(3-(4-Amino-3-(3-chloro-4-(pyidin-2-ylmethoxy)phenyl)-1H-pyrazolo[3,4-d]pyrimidin-1-yl)piperidin-1-yl)prop-2-en-1-one (CHMFL-EGFR-202) as a Novel Irreversible EGFR Mutant Kinase Inhibitor with a Distinct Binding Mode", JOURNAL OF MEDICINAL CHEMISTRY, 2017, 60, 7, 2944-2962.
(23) WOLLE PATRIK et al., "Characterization of Covalent Pyrazolopyrimidine-MKK7 Complexes and a Report on a Unique DFG-in/Leu-in Conformation of Mitogen-Activated Protein Kinase Kinase 7 (MKK7)", JOURNAL OF MEDICINAL CHEMISTRY, vol. 62, no. 11, 14 May 2019 (2019-05-14), pages 5541-5546.
(24) YIN YUAN et al., "Discovery of novel selective Janus kinase 2 (JAK2) inhibitors bearing a 1H-pyrazolo[3,4-d]pyrimidin-4-amino scaffold", BIOORGANIC & MEDICINAL CHEMISTRY, vol. 27 , no.8, 1 April 2019 (2019-04-01), pages 1562-1576, XP093078061, AMSTERDAM, NL ISSN: 0968-0896.
(25) ENGEL JULIAN et al., "Structure-Guided Development of Covalent and Mutant-Selective Pyrazolopyrimidines to Target T790M Drug Resistance in Epidermal Growth Factor Receptor", JOURNAL OF MEDICINAL CHEMISTRY, vol. 60, no. 18, 18 September 2017 (2017-09-18), pages 7725-7744, XP093078033, US ISSN: 0022-262.
(26) YUMING WANG et al., "Discovery of Potent lrreversible Pan-Fibroblast Growth Factor Receptor (FGFR) Inhibitors", JOURNAL OF MEDICINAL CHEMISTRY, vol. 61, no. 20, 9 March 2018 (2018-03-09), pages 9085-9104.
(27) HE, Linhong et al., "Design, synthesis and biological evaluation of 7H-pyrrolo[2,3-dlpyrimidin-4-amine derivatives as selective Btk inhibitors with improved pharmacokinetic properties for the treatment of arthritis", European Journal of Medicinal Chemistry, vol. 145, 2018, pages 96-112.

## Claims

1. A compound of formula I or a pharmaceutically acceptable salt thereof: 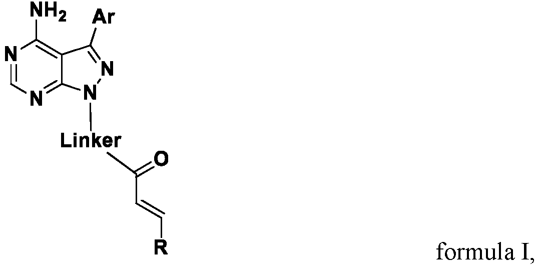
wherein the Ar is 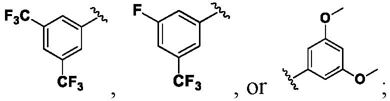
wherein the R is H, CF₃, (CH₃)₂NCH₂, or CH₃; and
wherein the Linker is 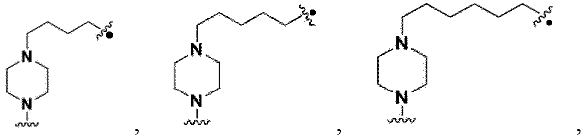 or 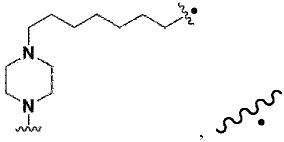 symbol representing a site bonding to N atom;
provided that the compound is not

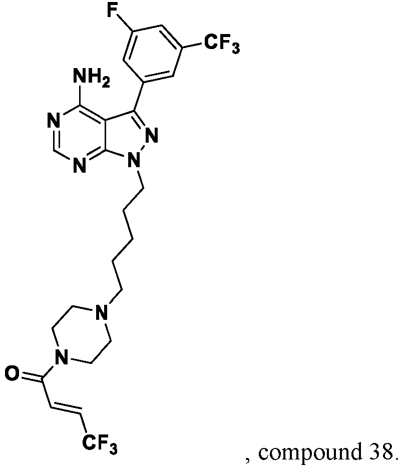

2. The compound or the pharmaceutically acceptable salt thereof according to claim 1, wherein the compound is one selected from the group consisting of the following compounds: 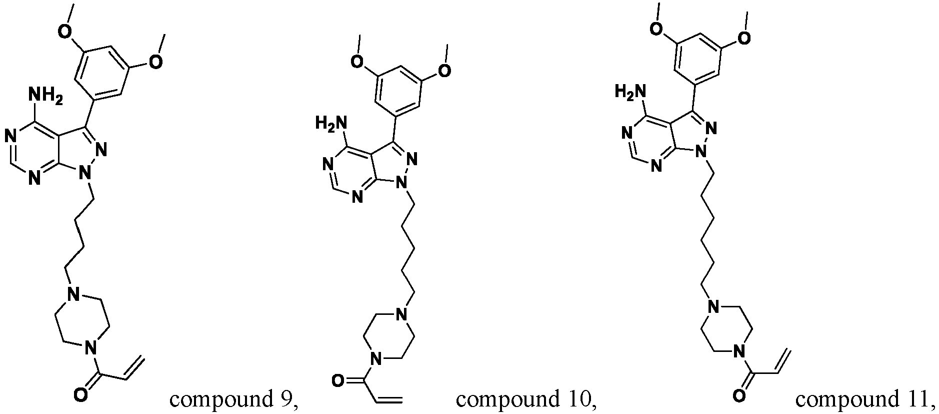 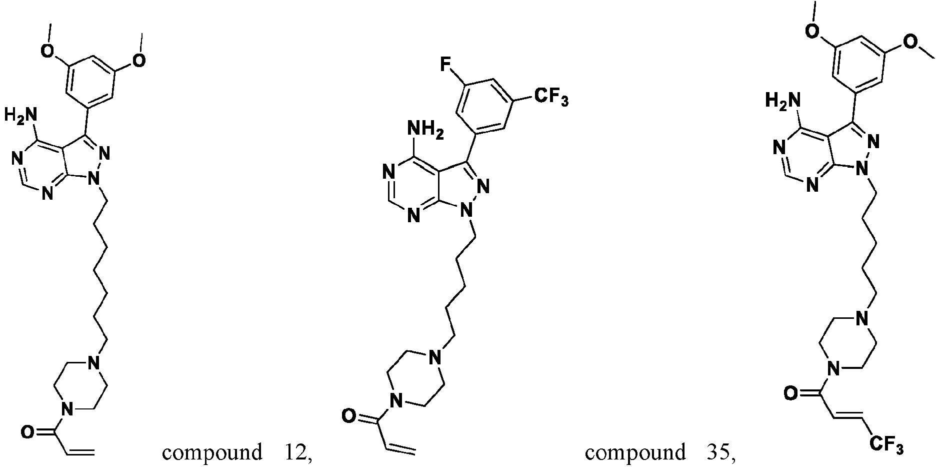 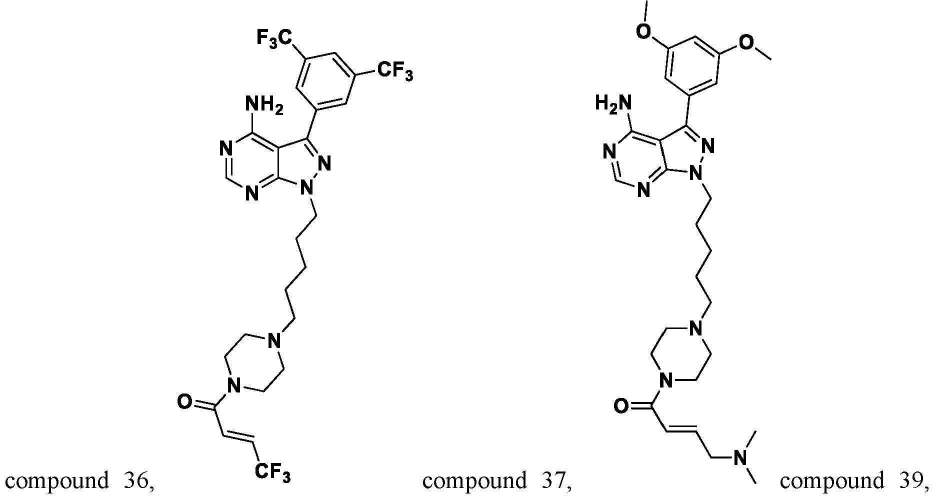 and

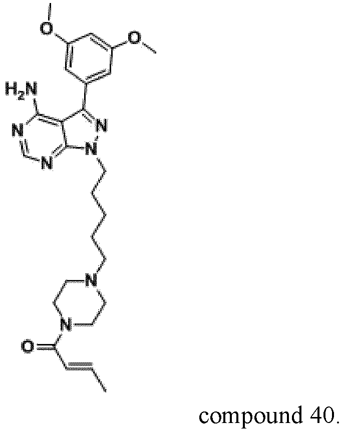

3. A method for preparing the compound or the pharmaceutically acceptable salt thereof according to any one of claims 1 to 2, with a synthetic route as follows:

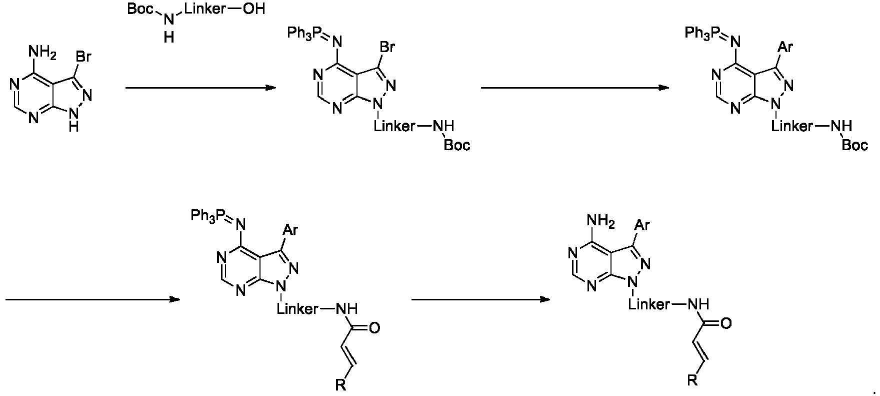

4. The method according to claim 3, comprising the following steps:
subjecting a compound with a structure shown in formula a and a compound with a structure shown in formula b to a Mitsunobu reaction at room temperature for 4 h to obtain a compound with a structure shown in formula c;
subjecting the compound with the structure shown in formula c and a compound with a structure shown in formula d to a Suzuki coupling reaction at 80°C to obtain a compound with a structure shown in formula e;
subjecting the compound with the structure shown in formula e and trifluoroacetic acid (TFA) to a deprotection reaction at 0°C to obtain a compound with a structure shown in formula f; and
subjecting the compound with the structure shown in formula f to an acylation reaction at room temperature to obtain the compound with a structure shown in formula I; 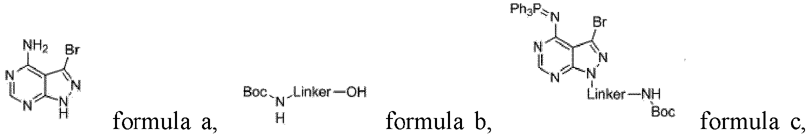
Ar-B(OH)₂ formula d,

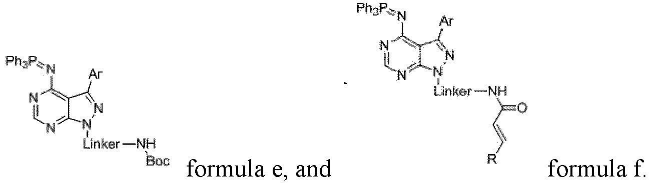

5. A pharmaceutical composition comprising the compound or the pharmaceutically acceptable salt thereof according to any one of claims 1 to 2, wherein the pharmaceutical composition further comprises one or more pharmaceutically acceptable excipients; and
a dosage form of the pharmaceutical composition is any pharmaceutically acceptable dosage form.

6. Compound or the pharmaceutically acceptable salt thereof according to any one of claims 1 to 2 or the pharmaceutical composition according to claim 5 for use in a method of treating and/or preventing a cancer.

7. The compound or the pharmaceutically acceptable salt thereof according to claims 1 to 2 or the pharmaceutical composition according to claim 5 for use according to claim 6, wherein the cancer is an FGFR abnormality-associated cancer.

8. The compound or the pharmaceutically acceptable salt thereof according to claims 1 to 2 or the pharmaceutical composition according to claim 5 for use according to claim 6, wherein the pharmaceutical composition is used as irreversible pan-FGFR inhibitor.

9. The compound or the pharmaceutically acceptable salt thereof according to any one of claims 1 to 2 or the pharmaceutical composition according to claim 5 for use according to claim 6, wherein the method comprises orally administrating or intraperitoneally injecting the compound or the pharmaceutically acceptable salt thereof according to any one of claims 1 to 2 or the pharmaceutical composition according to claim 5.

10. The compound or the pharmaceutically acceptable salt thereof according to any one of claims 1 to 2 or the pharmaceutical composition according to claim 5 for use according to claim 9, wherein the compound is compound 10 or the compound 36.

11. The compound or the pharmaceutically acceptable salt thereof according to any one of claims 1 to 2 or the pharmaceutical composition according to claim 5 for use according to claim 10, wherein the compound 10 or the compound 36 is administered at a dose of 50 mg/kg or 100 mg/kg.

## Patentansprüche

1. Verbindung der Formel I oder ein pharmazeutisch verträgliches Salz davon: 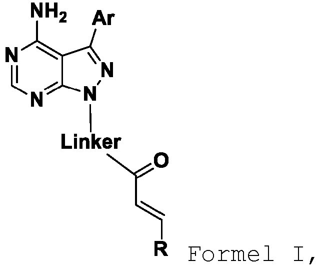
wobei Ar 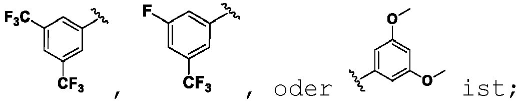
wobei R H, CF₃, (CH₃)₂NCH₂, oder CH₃ ist;
und wobei der Linker 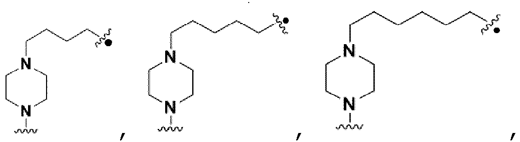 oder 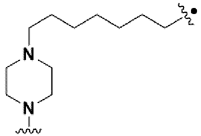 ist, wobei das 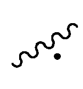 Symbol eine Stelle darstellt, die an ein N-Atom;
mit der Maßgabe, dass die Verbindung nicht 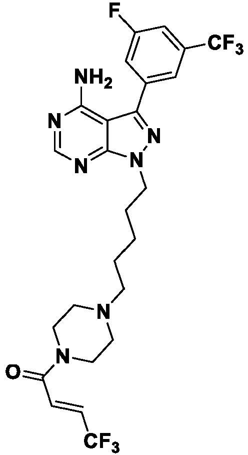 fiVerbindung 38 ist.

2. Die Verbindung oder ein pharmazeutisch verträgliches Salz davon gemäß Anspruch 1, wobei die Verbindung eine aus der Gruppe ausgewählt ist, die aus den folgenden Verbindungen besteht:

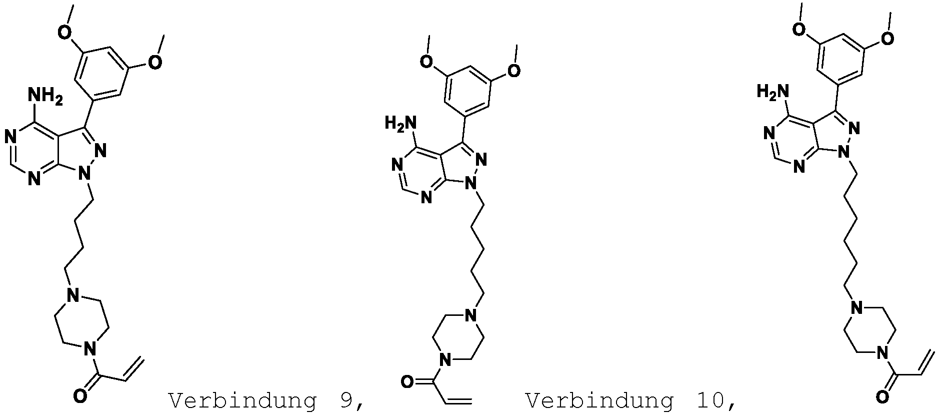

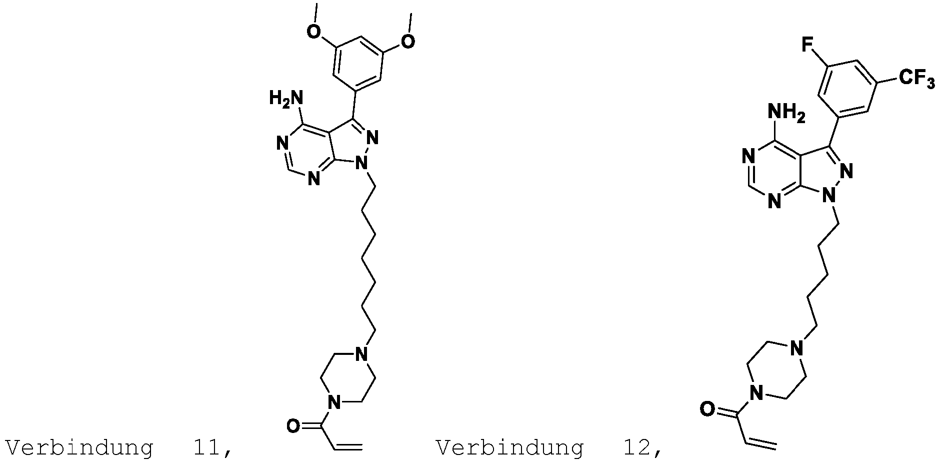

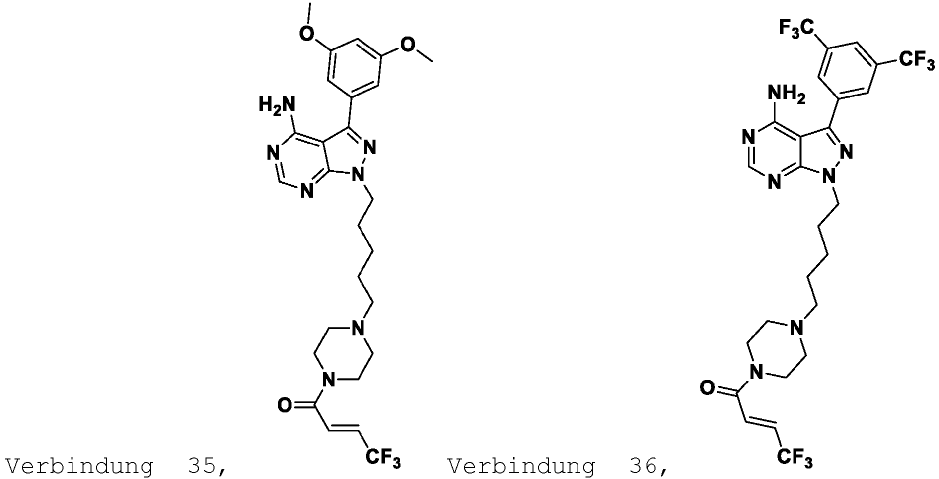

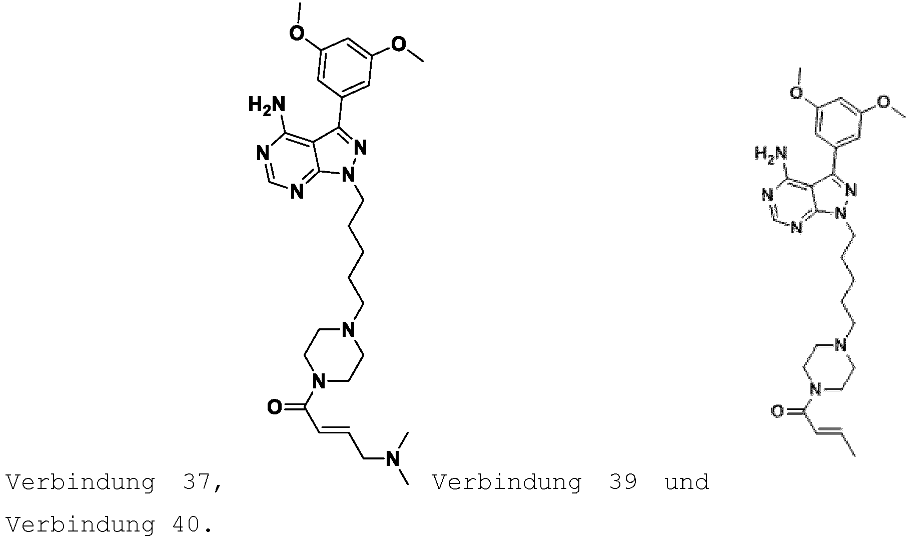

3. Verfahren zur Herstellung der Verbindung oder ihres pharmazeutisch verträglichen Salzes gemäß einem der Ansprüche 1 bis 2 mit folgendem Syntheseweg:

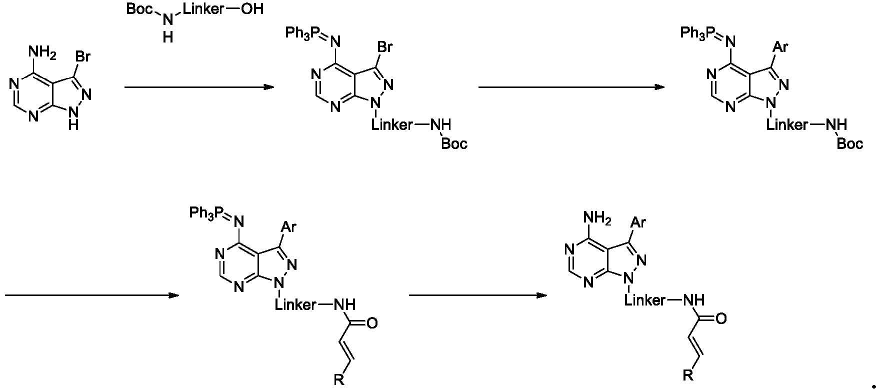

4. Verfahren nach Anspruch 3, umfassend die folgenden Schritte:
eine Verbindung mit einer Struktur gemäß Formel a und eine Verbindung mit einer Struktur gemäß Formel b einer Mitsunobu-Reaktion bei Raumtemperatur für 4 Stunden unterziehen, um eine Verbindung mit einer Struktur gemäß Formel c zu erhalten;
die Verbindung mit der in Formel c gezeigten Struktur und eine Verbindung mit einer in Formel d gezeigten Struktur einer Suzuki-Kupplungsreaktion bei 80 °C unterziehen, um eine Verbindung mit einer in Formel e gezeigten Struktur zu erhalten; die Verbindung mit der in Formel e gezeigten Struktur und Trifluoressigsäure (TFA) einer Entschützungsreaktion bei 0 °C unterziehen, um eine Verbindung mit einer in Formel f gezeigten Struktur zu erhalten; und
die Verbindung mit der in Formel f gezeigten Struktur einer Acylierungsreaktion bei Raumtemperatur unterziehen, um die Verbindung mit der in Formel I gezeigten Struktur zu erhalten; 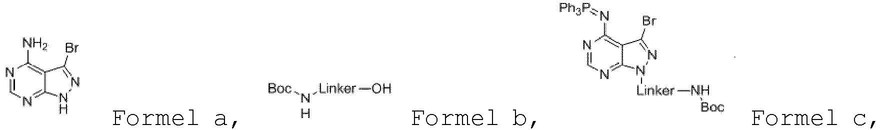
Ar- B(OH)₂ Formel d,

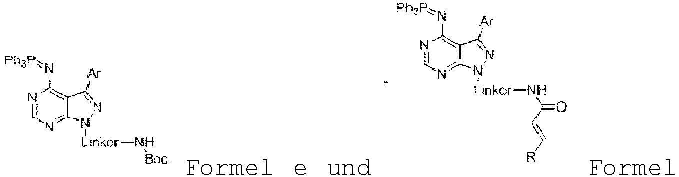

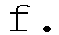

5. Pharmazeutische Zusammensetzung, umfassend die Verbindung oder das pharmazeutisch verträgliche Salz davon gemäß einem der Ansprüche 1 bis 2, wobei die pharmazeutische Zusammensetzung ferner einen oder mehrere pharmazeutisch verträgliche Hilfsstoffe umfasst; und
eine Darreichungsform der pharmazeutischen Zusammensetzung ist eine beliebige pharmazeutisch verträgliche Darreichungsform.

6. Verbindung oder deren pharmazeutisch verträgliches Salz gemäß einem der Ansprüche 1 bis 2 oder die pharmazeutische Zusammensetzung gemäß Anspruch 5 zur Verwendung in einem Verfahren zur Behandlung und/oder Vorbeugung von Krebs.

7. Die Verbindung oder deren pharmazeutisch verträgliches Salz gemäß den Ansprüchen 1 bis 2 oder die pharmazeutische Zusammensetzung gemäß Anspruch 5 zur Verwendung gemäß Anspruch 6, wobei der Krebs ein mit einer FGFR-Anomalie assoziierter Krebs ist.

8. Verbindung oder pharmazeutisch verträgliches Salz davon gemäß einem der Ansprüche 1 bis 2 oder pharmazeutische Zusammensetzung gemäß Anspruch 5 zur Verwendung gemäß Anspruch 6, wobei die pharmazeutische Zusammensetzung als irreversibler Pan-FGFR-Inhibitor verwendet wird.

9. Die Verbindung oder das pharmazeutisch verträgliche Salz davon gemäß einem der Ansprüche 1 bis 2 oder die pharmazeutische Zusammensetzung gemäß Anspruch 5 zur Verwendung gemäß Anspruch 6, wobei das Verfahren die orale Verabreichung oder intraperitonealeInjektion der Verbindung oder des pharmazeutisch verträglichen Salzes davon gemäß einem der Ansprüche 1 bis 2 oder der pharmazeutischen Zusammensetzung gemäß Anspruch 5 umfasst.

10. Die Verbindung oder das pharmazeutisch verträgliche Salz davon gemäß einem der Ansprüche 1 bis 2 oder die pharmazeutische Zusammensetzung gemäß Anspruch 5 zur Verwendung gemäß Anspruch 9, wobei die Verbindung die Verbindung 10 oder die Verbindung 36 ist.

11. Die Verbindung oder das pharmazeutisch verträgliche Salz davon gemäß einem der Ansprüche 1 bis 2 oder die pharmazeutische Zusammensetzung gemäß Anspruch 5 zur Verwendung gemäß Anspruch 10, wobei die Verbindung 10 oder die Verbindung 36 in einer Dosis von 50 mg/kg oder 100 mg/kg verabreicht wird.

## Revendications

1. Composé de formule I ou sel pharmaceutiquement acceptable de celui-ci : 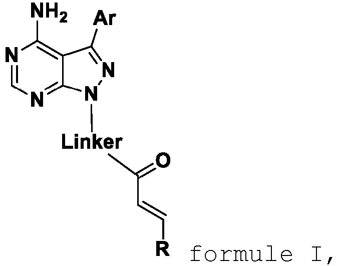
dans lequel Ar est 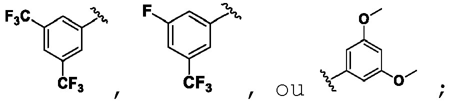
dans lequel R est H, CF₃, (CH₃)₂NCH₂ ou CH₃ ; et
dans lequel le lieur est 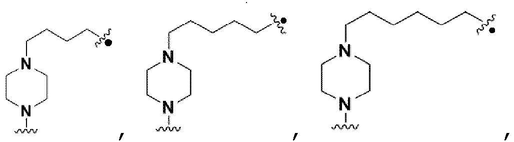 ou 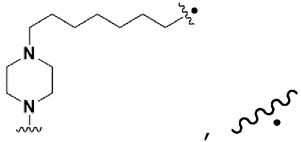 symbole représentant un site se liant à l'atome N ;
à condition que le composé ne soit pas

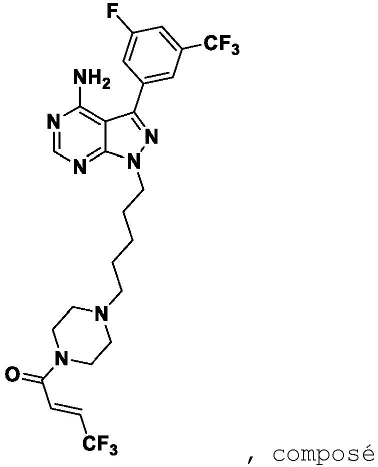

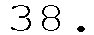

2. Composé ou sel pharmaceutiquement acceptable de celui-ci selon la revendication 1, dans lequel le composé est choisi dans le groupe constitué des composés suivants :

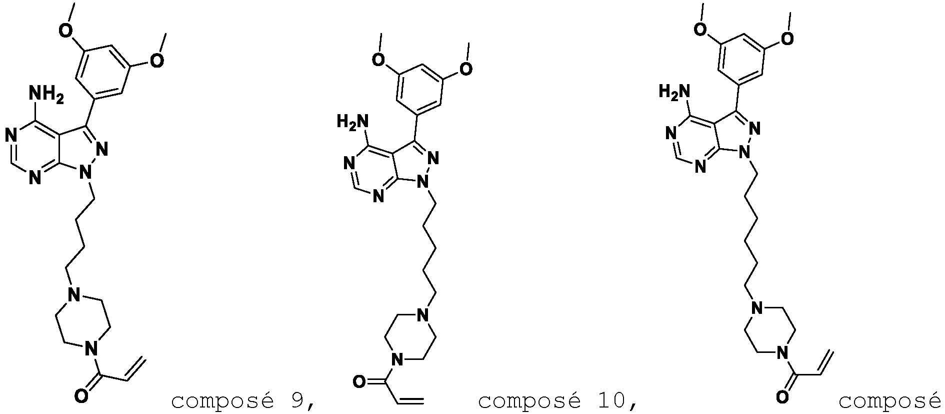

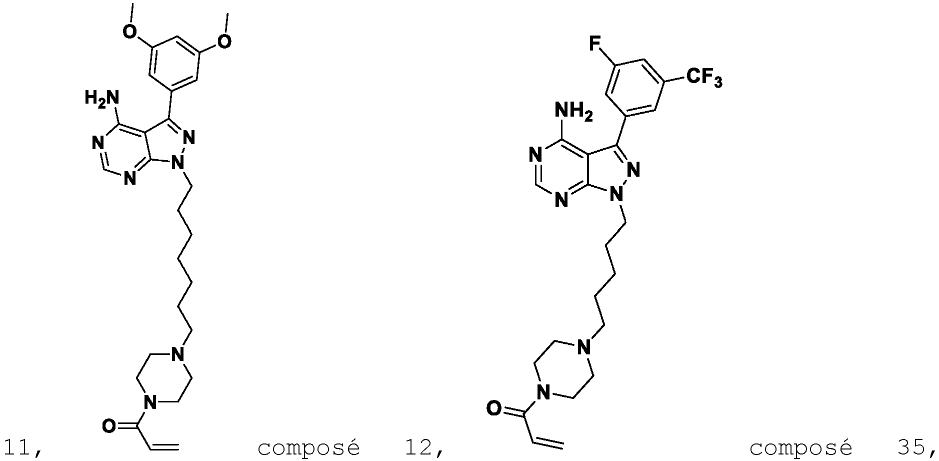

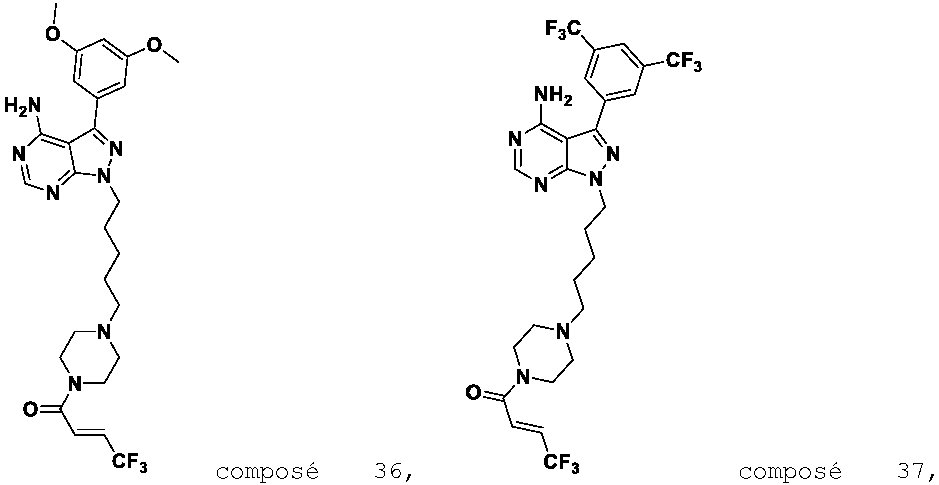

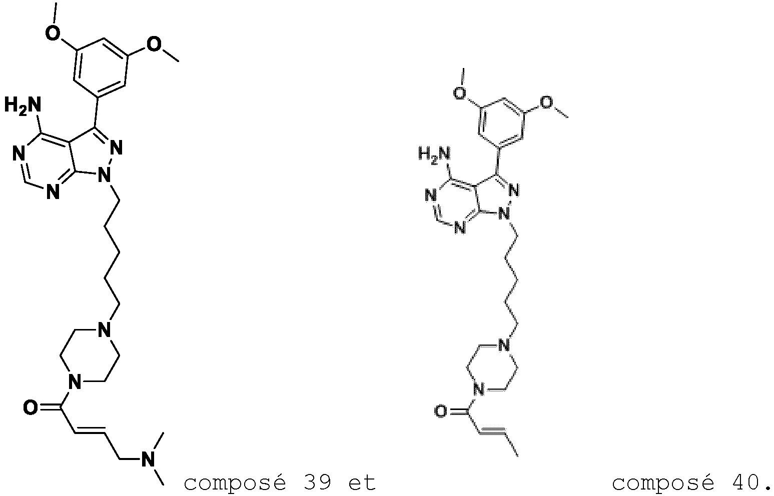

3. Procédé de préparation du composé ou du sel pharmaceutiquement acceptable de celui-ci selon l'une quelconque des revendications 1 à 2, avec une voie de synthèse comme suit :

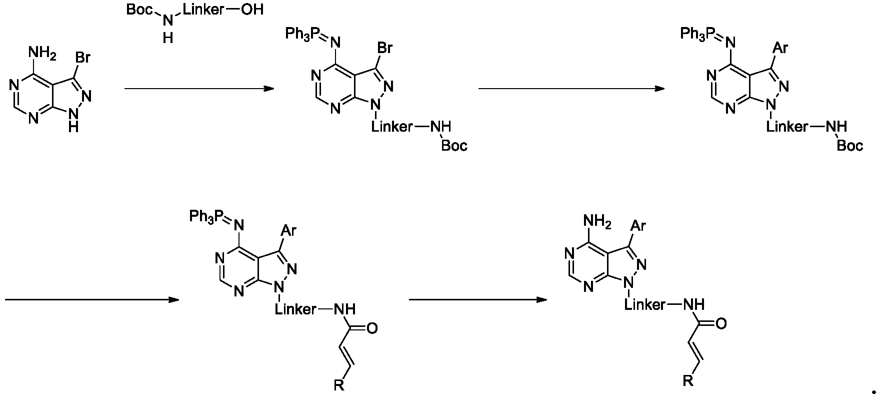

4. Procédé selon la revendication 3, comprenant les étapes suivantes :
soumission d'un composé avec une structure représentée dans la formule a et d'un composé avec une structure représentée dans la formule b à une réaction de Mitsunobu à température ambiante pendant 4 heures pour obtenir un composé avec une structure représentée dans la formule c ;
soumission du composé avec la structure représentée dans la formule c et d'un composé avec la structure représentée dans la formule d à une réaction de couplage de Suzuki à 80 °C pour obtenir un composé avec une structure représentée dans la formule e ;
soumission du composé avec la structure représentée dans la formule e et de l'acide trifluoroacétique (TFA) à une réaction de déprotection à 0 °C pour obtenir un composé avec une structure représentée dans la formule f ; et
soumission du composé avec la structure représentée dans la formule f à une réaction d'acylation à température ambiante pour obtenir le composé avec une structure représentée dans la formule I ; 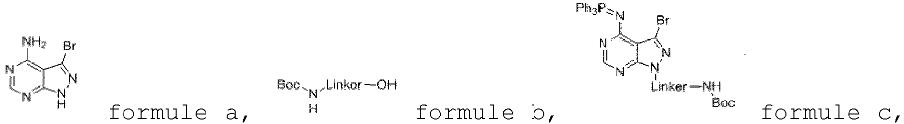
Ar-B(OH)₂ formule d,

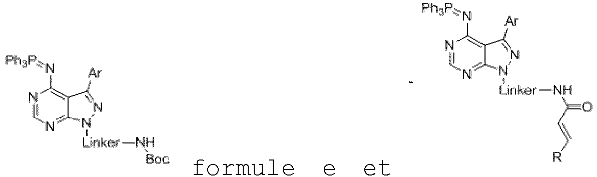

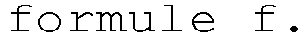

5. Composition pharmaceutique comprenant le composé ou le sel pharmaceutiquement acceptable de celui-ci selon l'une quelconque des revendications 1 à 2, dans lequel la composition pharmaceutique comprend également un ou plusieurs excipients pharmaceutiquement acceptables ; et
une forme posologique de la composition pharmaceutique est toute forme posologique pharmaceutiquement acceptable.

6. Composé ou sel pharmaceutiquement acceptable de celui-ci selon l'une quelconque des revendications 1 à 2 ou composition pharmaceutique selon la revendication 5 pour une utilisation dans un procédé de traitement et/ou de prévention d'un cancer.

7. Composé ou sel pharmaceutiquement acceptable de celui-ci selon les revendications 1 à 2 ou composition pharmaceutique selon la revendication 5 pour une utilisation selon la revendication 6, dans lequel le cancer est un cancer associé à une anomalie du FGFR.

8. Composé ou sel pharmaceutiquement acceptable de celui-ci selon les revendications 1 à 2 ou composition pharmaceutique selon la revendication 5 pour une utilisation selon la revendication 6, dans lequel la composition pharmaceutique est utilisée comme inhibiteur pan-FGFR irréversible.

9. Composé ou sel pharmaceutiquement acceptable de celui-ci selon l'une quelconque des revendications 1 à 2 ou composition pharmaceutique selon la revendication 5 pour une utilisation selon la revendication 6, dans lequel le procédé comprend l'administration orale ou l'injection intrapéritonéale du composé ou du sel pharmaceutiquement acceptable de celui-ci selon l'une quelconque des revendications 1 à 2 ou de la composition pharmaceutique selon la revendication 5.

10. Composé ou sel pharmaceutiquement acceptable de celui-ci selon l'une quelconque des revendications 1 à 2 ou composition pharmaceutique selon la revendication 5 pour une utilisation selon la revendication 9, dans lequel le composé est le composé 10 ou le composé 36.

11. Composé ou sel pharmaceutiquement acceptable de celui-ci selon l'une quelconque des revendications 1 à 2 ou composition pharmaceutique selon la revendication 5 pour une utilisation selon la revendication 10, dans lequel le composé 10 ou le composé 36 est administré à une dose de 50 mg/kg ou 100 mg/kg.
